(19) Europäisches Patentamt
European Patent Office
Office européen des brevets

(11) EP 1 491 353 A1

(12) EUROPEAN PATENT APPLICATION
published in accordance with Art. 158(3) EPC

(43) Date of publication:
**29.12.2004 Bulletin 2004/53**

(21) Application number: **03715517.3**

(22) Date of filing: **27.03.2003**

(51) Int Cl.[7]: **B41M 5/26**, C09B 23/04, C07D 209/70, C07D 491/048, C07D 495/04, G11B 7/24

(86) International application number:
**PCT/JP2003/003840**

(87) International publication number:
**WO 2003/082593 (09.10.2003 Gazette 2003/41)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **29.03.2002 JP 2002096351**
**02.10.2002 JP 2002290155**

(71) Applicants:
- **MITSUI CHEMICALS, INC.**
  **Tokyo (JP)**
- **Yamamoto Chemicals, Inc.**
  **Yao-shi, Osaka 581-0034 (JP)**

(72) Inventors:
- **NISHIMOTO, Taizo, Mitsui Chemicals, Inc.**
  **Sodegaura-shi, Chiba 299-0265 (JP)**
- **INOUE, Shinobu**
  **Sodegaura-shi, Chiba 299-0265 (JP)**
- **MISAWA, Tsutami**
  **Sodegaura-shi, Chiba 299-0265 (JP)**
- **NARA, Ryosuke**
  **Sodegaura-shi, Chiba 299-0265 (JP)**
- **INATOMI, Yuji**
  **Sodegaura-shi, Chiba 299-0265 (JP)**
- **MURAYAMA, Shunsuke**
  **Sodegaura-shi, Chiba 299-0265 (JP)**
- **KOIKE, Tadashi**
  **Sodegaura-shi, Chiba 299-0265 (JP)**
- **SAITO, Yasunori**
  **Sodegaura-shi, Chiba 299-0265 (JP)**
- **NAKAGAWA, Shinichi**
  **Sodegaura-shi, Chiba 299-0265 (JP)**

(74) Representative: **Stuart, Ian Alexander et al**
**Mewburn Ellis LLP**
**York House**
**23 Kingsway**
**London WC2B 6HP (GB)**

# (54) OPTICAL RECORDING MEDIA AND DIPYRROMETHENE-METAL CHELATE COMPOUNDS

(57) An optical recording medium containing in the recording layer a dipyrromethene-metal chelate compound of which the initial principal weight loss temperature is thermogravimetrically from 330°C through 500°C inclusive, capable of high speed and high density recording and playback with a laser having a wavelength of 520 to 690 nm.

Figure 1

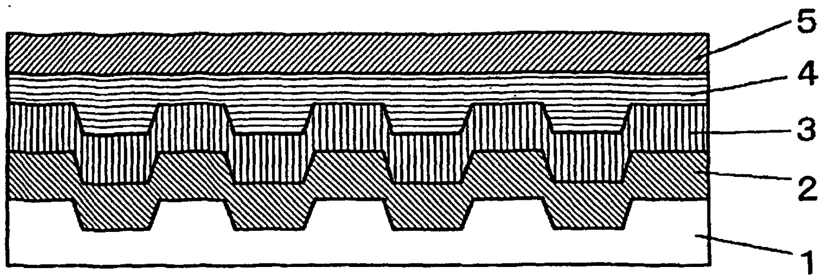


EP 1 491 353 A1

## Description

Technical Field

The present invention relates to a dipyrromethene-metal chelate compound, and a DVD-compatible recordable optical recording medium using the same that can record at high speed.

Background Art

Among disc media that record using light, recordable compact discs that are able to record only once are termed CD-R. These discs are now used by a lot of people, as they are compatible with an ordinary read-only CD-ROM. Furthermore, development of recording media for digital versatile discs (DVD), which have a higher recording density than a CD, has been progressing, wherein widespread use of recordable DVD-R having a single-side 4.7 GB recording capacity that can fit a motion picture recording with TV quality using a red semiconductor laser having a oscillating wavelength of from 635 nm to 660 nm is expected because of a high compatibility with DVD-ROM.

These recordable media are able to record and regenerate information by forming pits that produce a change in the reflectance, by causing chemical or physical change in the organic dye of the recording layer, such as decomposition, evaporation or dissolution, through the absorption of irradiated laser beam energy.

Various dyes have been proposed and put into practical use as dyes to be used in DVD-R recording layer, such as cyanine dyes, azo metal chelate dyes, tetraazaporphyrin dyes and porphyrin dyes. The inventors of the present invention have focused their attention on dipyrromethene-metal chelate compounds, which have excellent optical properties, recording characteristics and durability. Starting with Japanese Patent Laid-Open No. 10-226172 and Japanese Patent Laid-Open No. 11-092682, they have put forward a large number of optical recording media that use this line of dyes.

However, as the recording speeds become faster, such as 4x-speed recording, it becomes more difficult to control pit formation of the organic dye in the recording layer, giving rise to problems of deteriorating recording characteristics such as jitter and error incidence. It also becomes difficult to simultaneously satisfy the recording characteristics for standard-speed recording with those for high-speed recording. This creates the problem that in order to satisfy the characteristics for one of these, it becomes necessary to sacrifice the characteristics for the other.

Disclosure of the Invention

It is an object of the present invention to provide an optical recording medium that uses dipyrromethene-metal chelate compound as an optical recording medium that solves the above-described problems.

As a result of intensive investigation to achieve this object, the inventors found that, as an organic dye to be used in a recording layer, an optical recording medium excellent in not only standard recording but also high-speed recording such as 4x-speed recording can be obtained by using a dipyrromethene-metal chelate compound of which the initial principal weight loss temperature is thermogravimetrically from 330°C through 500°C inclusive, in particular using a dipyrromethene-metal chelate compound represented by the general formula (1), thereby arriving at the present invention.

That is, the present invention is:

1. An optical recording medium comprising at least a recording layer and a reflecting layer on a transparent substrate having a guide groove formed thereon, wherein the recording layer contains at least one dipyrromethene-metal chelate compound in which initial principal weight loss temperature according to thermogravimetric analysis is from 330°C or more to 500°C or less.
2. The optical recording medium according to the above item 1, wherein the dipyrromethene-metal chelate compound is represented by general formula (1),

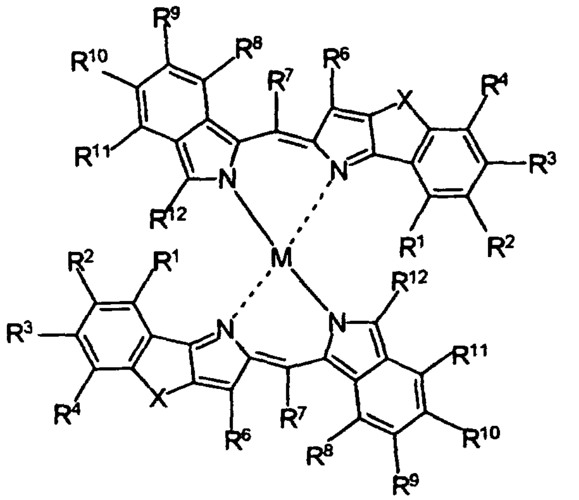

(1)

wherein $R^1$ to $R^4$ and $R^6$ to $R^{11}$ each independently represent hydrogen, halogen, optionally substituted alkyl, alkoxy, aryl, aryloxy or a substituent represented by formula (a);

$$-CO-R^{13} \qquad (a)$$

(wherein $R^{13}$ represents optionally substituted alkyl, optionally substituted aralkyl or optionally substituted aryl)
$R^{12}$ represents optionally substituted aryl;
X represents -O-, -S- or -CH($R^5$)-; $R^5$ represents hydrogen, halogen, optionally substituted alkyl, alkoxy, or aryl; and M represents transition element.
3. The optical recording medium according to the above item 2, wherein the dipyrromethene-metal chelate compound is represented by general formula (2),

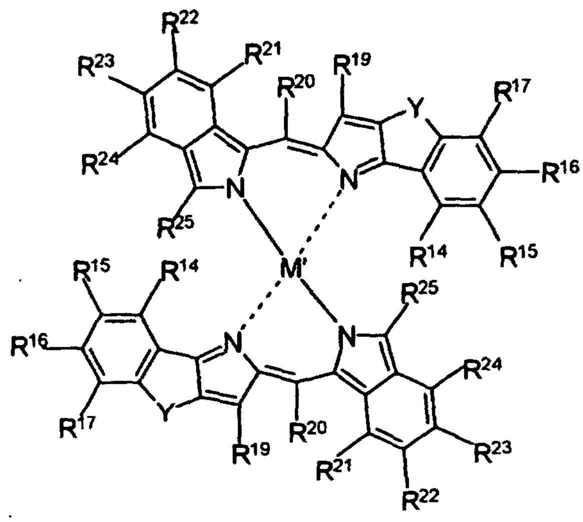

(2)

wherein $R^{14}$ to $R^{17}$ and $R^{19}$ to $R^{24}$ each independently represent hydrogen, halogen, optionally substituted alkyl, alkoxy, aryl or aryloxy; $R^{25}$ represents optionally substituted aryl; Y represents -O-, -S- or -CH($R^{18}$)-; $R^{18}$ represents hydrogen, halogen, optionally substituted alkyl, alkoxy or aryl; and M' represents transition element.
4. The optical recording medium according to the above item 1, comprising a mixture of dipyrromethene-metal chelate compounds represented by general formulas (3), (4) and (5),

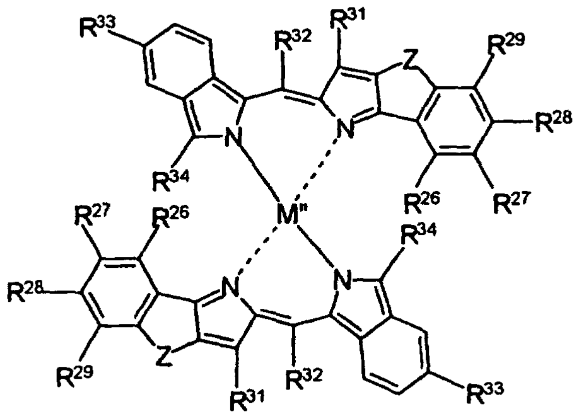

(3)

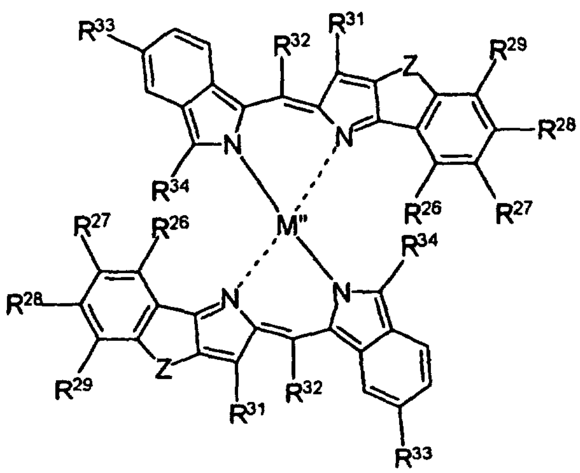

(4)

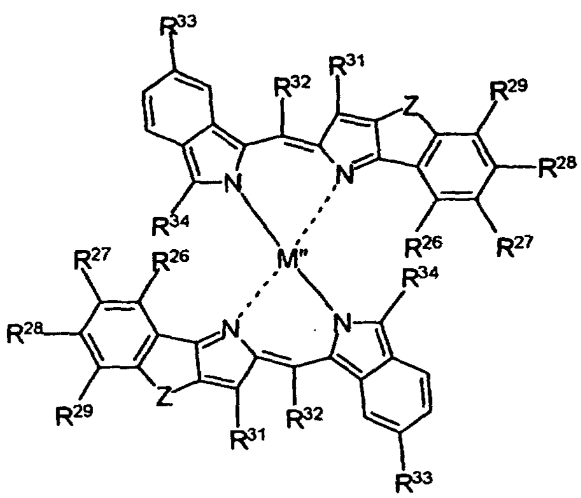

(5)

wherein $R^{26}$ to $R^{29}$ and $R^{31}$ to $R^{32}$ each independently represent hydrogen, halogen, optionally substituted alkyl, alkoxy, aryl, aryloxy or a substituent represented by formula (b);

$$- CO-R^{35} \quad \text{(b)}$$

(wherein $R^{35}$ represents optionally substituted alkyl, optionally substituted aralkyl or optionally substituted aryl) $R^{33}$ represents halogen, optionally substituted alkyl, alkoxy, aryl or aryloxy; $R^{34}$ represents optionally substituted aryl; Z represents -O-, -S- or -CH-$R^{30}$-; $R^{30}$ represents hydrogen, halogen, optionally substituted alkyl, alkoxy, or aryl; and M" represents transition element.

5. A dipyrromethene-metal chelate compound represented by general formula (6),

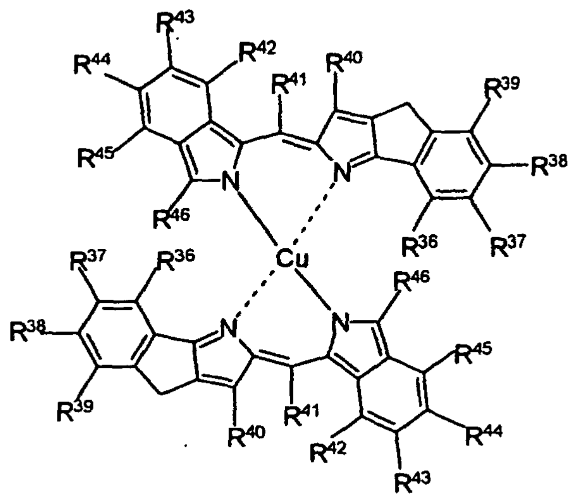

(6)

wherein $R^{36}$ to $R^{39}$ and $R^{41}$ to $R^{45}$ each independently represent hydrogen, halogen, optionally substituted alkyl, alkoxy, aryl, aryloxy; $R^{40}$ represents optionally substituted alkyl having a total number of from 2 to 12 carbon atoms; and $R^{46}$ represents optionally substituted aryl.

6. A mixture of dipyrromethene-metal chelate compounds represented by general formulas (7), (8) and (9),

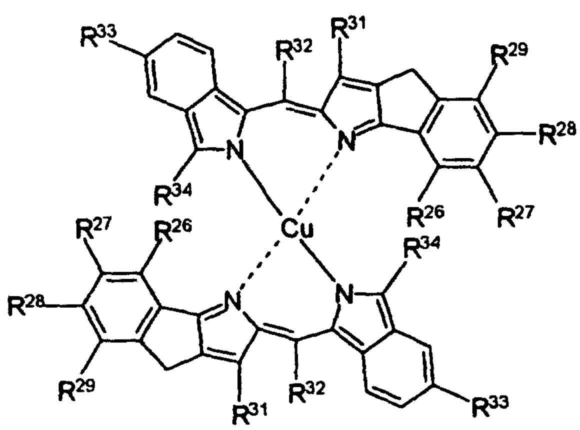

(7)

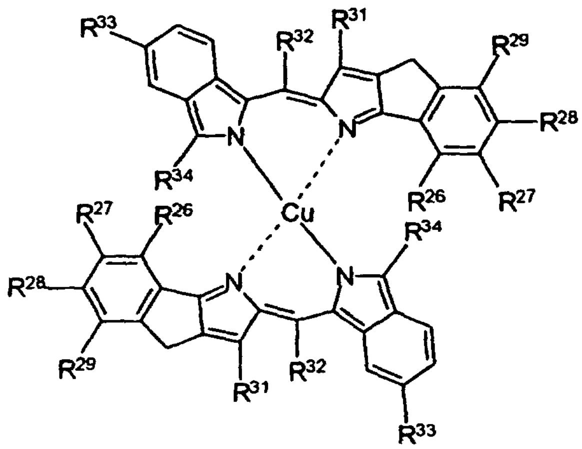

(8)

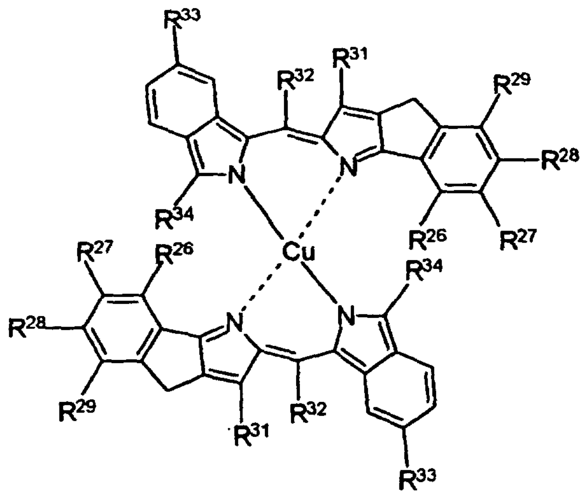

(9)

wherein $R^{26}$ to $R^{29}$ and $R^{31}$ to $R^{32}$ each independently represent hydrogen, halogen, optionally substituted alkyl, alkoxy, aryl, aryloxy or a substituent represented by formula (b);

$$-CO-R^{35} \qquad (b)$$

(wherein $R^{35}$ represents optionally substituted alkyl, optionally substituted aralkyl or optionally substituted aryl) $R^{33}$ represents halogen, optionally substituted alkyl, alkoxy, aryl or aryloxy; and $R^{34}$ represents optionally substituted aryl.

Brief Description of the Drawings

Figure 1 is a cross-sectional structural drawing illustrating layer structures in optical recording media according to the prior art and the present invention; and

Figure 2 is an example of a TG curve to explain initial thermal weight loss temperature, heat weight loss slope and total heat weight loss.

Best Mode for Carrying Out the Invention

The present invention will now be described in detail.

In the dipyrromethene metal chelate compound represented by the general formula (1), specific examples of the halogen atom of $R^1$ to $R^4$, and $R^6$ to $R^{11}$ include fluorine, chlorine, bromine, iodine and the like, and a bromine atom is especially preferable.

The optionally substituted alkyl group preferably has a total of from 1 to 12 carbon atoms, and may be a linear chain, branched, or annular.

Examples thereof include primary alkyls such as methyl, ethyl, n-propyl, n-butyl, n-pentyl, n-hexyl, n-heptyl, n-octyl, n-nonyl and n-decyl; secondary alkyls such as isobutyl, isoamyl, 2-methylbutyl, 2-methylpentyl, 3-methylhexyl, 4-methylhexyl, 5-methylhexyl, 2-ethylpentyl, 3-ethylpentyl, 2-methylheptyl, 3-methylheptyl, 4-methylheptyl, 5-methylheptyl, 2-ethylhexyl, 3-ethylhexyl, isopropyl, sec-butyl, 1-ethylpropyl, 1-methylbutyl, 1,2-dimethylpropyl, 1-methylheptyl, 1-ethylbutyl, 1,3-dimethylbutyl, 1,2-dimethylbutyl, 1-ethyl-2-methylpropyl, 1-methylhexyl, 1-ethylheptyl, 1-propylbutyl, 1-isopropyl-2-methylpropyl, 1-ethyl-2-methylbutyl, 1-propyl-2-methylpropyl, 1-methylheptyl, 1-ethylhexyl, 1-propylpentyl, 1-isopropylpentyl, 1-isopropyl-2-methylbutyl, 1-isopropyl-3-methylbutyl, 1-methyloctyl, 1-propylhexyl and 1-isobutyl-3-methylbutyl; tertiary alkyls such as neopentyl, tert-butyl, tert-amyl, tert-hexyl and tert-octyl; and cycloalkyls such as cyclohexyl, 4-methylcyclohexyl, 4-ethylcyclohexyl, 4-tert-butylcyclohexyl, bornyl and isobornyl (adamantane radical).

The above-described alkyl group may be substituted with a halogen atom, and may also be substituted with the above-described alkyl group through an atom such as oxygen, sulfur and nitrogen. Examples of the alkyl group substituted through oxygen include methoxymethyl, methoxyethyl, ethoxymethyl, ethoxyethyl, butoxyethyl, ethoxyethoxyethyl, phenoxyethyl, methoxypropyl, ethoxypropyl, piperidino, morpholino and the like. Examples of the alkyl group substituted through sulfur include methylthioethyl, ethylthioethyl, ethylthiopropyl, phenylthioethyl and the like. Examples of the alkyl group substituted through nitrogen include dimethylaminoethyl, diethylaminoethyl, diethylaminopropyl and the like.

The optionally substituted alkoxy group preferably has a total of from 1 to 12 carbon atoms, in which an optionally substituted alkyl group may be directly bonded to the oxygen atom, wherein the alkyls described above can be cited as such an alkyl.

The optionally substituted aryl group preferably has a total of from 6 to 18 carbon atoms. Examples include phenyl, naphthyl, biphenyl, 2-fluorenyl, phenanthyrl, anthracenyl, terphenyl and the like.

Such aryl group may be substituted with hydroxyl, a halogen atom, nitro, carboxy, and cyano, and may also be substituted with the above-described alkyl group through an atom such as oxygen, sulfur, and nitrogen.

Examples of these substituted aryl groups include nitrophenyl, cyanophenyl, hydroxyphenyl, methylphenyl, dimethylphenyl, trimethylphenyl, ethylphenyl, diethylphenyl, triethylphenyl, n-propylphenyl, di(n-propyl)phenyl, tri(n-propyl)phenyl, iso-propylphenyl, di(iso-propyl)phenyl, tri(iso-propyl)phenyl, n-buthylphenyl, di(n-butyl)phenyl, tri(n-butyl)phenyl, iso-buthylphenyl, di(iso-butyl)phenyl, tri(iso-butyl)phenyl, sec-buthylphenyl, di(sec-butyl)phenyl, tri(sec-butyl)phenyl, t-buthylphenyl, di(t-butyl)phenyl, tri(t-butyl)phenyl, dimethyl-t-buthylphenyl, fluorophenyl, chlorophenyl, bromophenyl, iodophenyl, methoxyphenyl, ethoxyphenyl, trifluoromethylphenyl, N,N-dimethylaminophenyl, naphthyl, nitronaphthyl, cyanonaphthyl, hydroxynaphthyl, methylnaphthyl, fluoronaphthyl, chloronaphthyl, bromonaphthyl, iodonaphthyl, methoxynaphthyl, trifluoromethylnaphthyl, N,N-dimethylaminonaphthyl and the like.

The optionally substituted aryloxy group preferably has a total of from 6 to 18 carbon atoms, in which an optionally substituted aryl group may be directly bonded to the oxygen atom, wherein the aryls described above can be cited as such an aryl.

From the viewpoint of the degree of modulation and reflectance, more preferable examples include $R^6$ being an optionally substituted alkyl having a total of from 2 to 12 carbon atoms.

Examples thereof include primary alkyls such as ethyl, n-propyl, n-butyl, n-pentyl, n-hexyl, n-heptyl, n-octyl, n-nonyl and n-decyl; secondary alkyls such as isobutyl, isoamyl, 2-methylbutyl, 2-methylpentyl, 3-methylhexyl, 4-methylhexyl, 5-methylhexyl, 2-ethylpentyl, 3-ethylpentyl, 2-methylheptyl, 3-methylheptyl, 4-methylheptyl, 5-methylheptyl, 2-ethylhexyl, 3-ethylhexyl, isopropyl, sec-butyl, 1-ethylpropyl, 1-methylbutyl, 1,2-dimethylpropyl, 1-methylheptyl, 1-ethylbutyl, 1,3-dimethylbutyl, 1,2-dimethylbutyl, 1-ethyl-2-methylpropyl, 1-methylhexyl, 1-ethylheptyl, 1-propylbutyl, 1-isopropyl-2-methylpropyl, 1-ethyl-2-methylbutyl, 1-propyl-2-methylpropyl, 1-methylheptyl, 1-ethylhexyl, 1-propylpentyl, 1-isopropylpentyl, 1-isopropyl-2-methylbutyl, 1-isopropyl-3-methylbutyl, 1-methyloctyl, 1-propylhexyl and 1-isobutyl-3-methylbutyl; tertiary alkyls such as neopentyl, tert-butyl, tert-amyl, tert-hexyl and tert-octyl; and cycloalkyls such as cyclohexyl, 4-methylcyclohexyl, 4-ethylcyclohexyl, 4-tert-butylcyclohexyl, bornyl and isobornyl (adamantane radical).

The above-described alkyl group may be substituted with a halogen atom, and may also be substituted with the above-described alkyl group through an atom such as oxygen, sulfur and nitrogen. Examples of the alkyl group substituted through oxygen include methoxymethyl, methoxyethyl, ethoxymethyl, ethoxyethyl, butoxyethyl, ethoxyethoxyethyl, phenoxyethyl, methoxypropyl, ethoxypropyl, piperidino, morpholino and the like. Examples of the alkyl group substituted through sulfur include methylthioethyl, ethylthioethyl, ethylthiopropyl, phenylthioethyl and the like. Examples of the alkyl group substituted through nitrogen include dimethylaminoethyl, diethylaminoethyl, diethylaminopropyl and the like.

Examples of the optionally substituted aryl group for $R^{12}$ include the above-described optionally substituted aryl groups.

Examples of the halogen atom, and the optionally substituted alkyl group, alkoxy group, and aryl group for $R^5$ include the above-described halogens, and optionally substituted alkyl, alkoxy and aryl groups.

In the substituents represented in formula (a), $R^{13}$ represents an optionally substituted alkyl group, an optionally substituted aralkyl group or an optionally substituted aryl group. Examples thereof include those described above.

Examples of M include any transition element as long as it can form a chelate with a dipyrromethene compound, including the metals of Groups 8, 9, 10 (Group VIII), Group 11 (Group Ib), Group 12 (Group IIb), Group 3 (Group IIIa), Group 4 (Group IVa), Group 5 (Group Va), Group 6 (Group VIa) and Group 7 (Group VIIa). Specifically, Cu(II), Zn(II), Ni(II), Pd(II), Pt(II), Mn(II) or Co(II), preferably Cu(II), Zn(II) or Pd(II), and Cu(II) is particularly preferable.

A dipyrromethene-metal chelate compound according to the present invention represented by general formula (1) may be, for example, prepared as described in, but not limited to, Aust. J. Chem, 1965, 11, 1835-45, Heteroatom Chemistry, Vol. 1, 5,389(1990), U.S. Patent No. 4,774,339 or U.S. Patent No. 5,433,896. It may be typically prepared by the following two-step reaction.

In the first step, a compound represented by general formula (10) is reacted with a compound represented by general formula (11), or a compound represented by general formula (12) is reacted with a compound represented by general formula (13), in the presence of an acid catalyst such as hydrobromic acid and hydrochloric acid in an appropriate solvent, to give a dipyrromethene compound represented by general formula (14). Then, in the second step, the dipyrromethene compound represented by general formula (14) is reacted with an acetate or halide of a transition metal, to give the dipyrromethene-metal chelate compound represented by general formula (1):

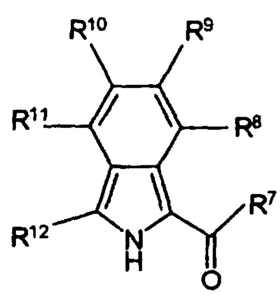

(10)

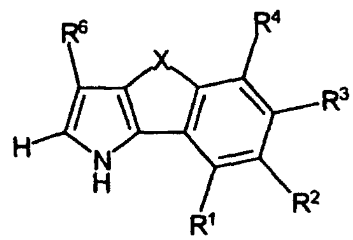

(11)

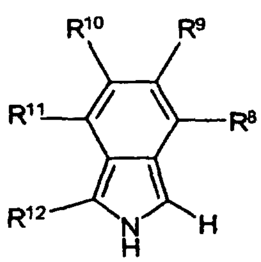

(12)

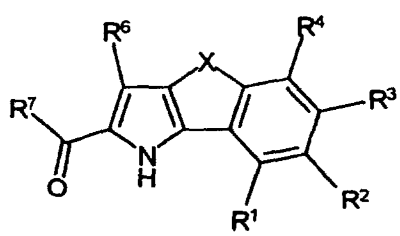

(13)

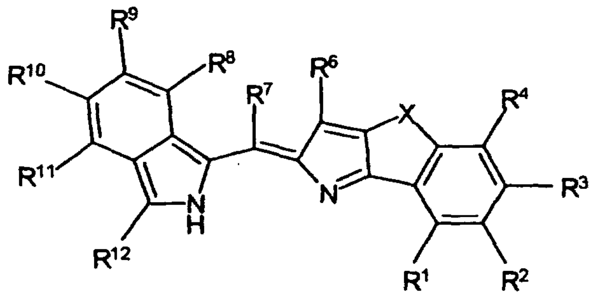

(14)

wherein in formulas (10) to (14), $R^1$ to $R^4$, $R^6$ to $R^{12}$ and X are as defined above.

The compound represented by general formula (11) may be, for example, prepared as described in Journal Organic Chemistry, 65, 2900(2000) or Journal Heterocyclic Chemistry, 30, 477(1993) and the like. The compound represented by general formula (13) may be prepared by acylating the compound represented by general formula (11), for example, according to a method described in Organic Preparations and Procedures Int. 13(2), 97-101(1981), J.O. C. 28, 3052-3058(1963) or Tetrahedron Letters 2411- (1989) and the like.

Table-1 shows preferable examples of a dipyrromethene metal chelate compound represented by general formula (1) of the present invention. When the dipyrromethene metal chelate compound has a group represented by formula (a) as a substituent (a-1 to a-5), the group represented by $R^{13}$ is shown in Table-2.

Table-1(1)

| Comp. | $R^1$ | $R^2$ | $R^3$ | $R^4$ | X | $R^6$ | $R^7$ | $R^8$ | $R^9$ | $R^{10}$ | $R^{11}$ | $R^{12}$ | M |
|---|---|---|---|---|---|---|---|---|---|---|---|---|---|
| 1−1 | H | H | H | H | S | H | H | H | H | $CH_3$ | H | 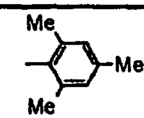 | Cu |
| 1−2 | H | H | H | H | O | H | H | H | H | Br | H | 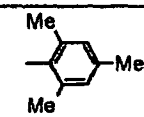 | Cu |
| 1−3 | H | H | H | H | O | H | H | H | H | $CH_3$ | H | 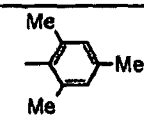 | Cu |
| 1−4 | H | H | H | H | O | H | H | H | Br | H | H | 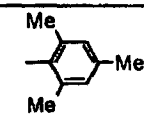 | Cu |
| 1−5 | H | H | H | H | $CH_2$ | $C_2H_5$ | H | H | H | $CH_3$ | H | 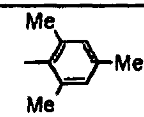 | Cu |
| 1−6 | H | H | H | H | $CH_2$ | $C_2H_5$ | H | H | H | $CH_3$ | H | 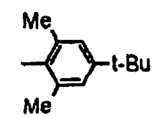 | Cu |
| 1−7 | H | H | H | H | $CH_2$ | $CH_3$ | H | H | H | Br | H | 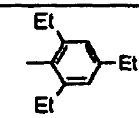 | Cu |
| 1−8 | H | H | H | H | $CH_2$ | $CH_3$ | H | H | H | $CH_3$ | H | 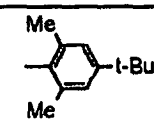 | Cu |

Table-1(2)

| Comp. | $R^1$ | $R^2$ | $R^3$ | $R^4$ | X | $R^6$ | $R^7$ | $R^8$ | $R^9$ | $R^{10}$ | $R^{11}$ | $R^{12}$ | M |
|---|---|---|---|---|---|---|---|---|---|---|---|---|---|
| 1−9 | H | H | H | H | $CH_2$ | $C_2H_5$ | H | H | H | Br | H | 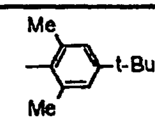 | Cu |
| 1−10 | H | H | H | H | $CH_2$ | $CH_3$ | H | H | H | Br | H | 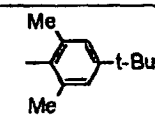 | Cu |
| 1−11 | H | H | H | H | $CHCH_3$ | $CH_3$ | H | H | H | $CH_3$ | H | 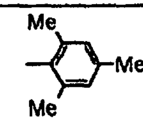 | Cu |
| 1−12 | H | H | H | H | $CH_2$ | H | H | H | H | H | H | 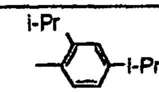 | Co |
| 1−13 | H | H | H | H | $CH_2$ | Br | H | H | Br | H | H | 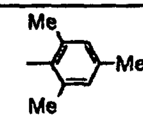 | Zn |
| 1−14 | H | H | H | H | O | H | H | Br | Br | Br | Br | 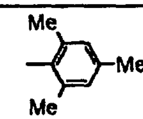 | Cu |
| 1−15 | H | H | H | H | O | H | H | H | H | Br | H | 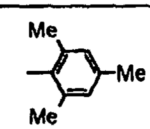 | Co |
| 1−16 | H | H | $OCH_3$ | H | S | H | H | H | H | H | H | 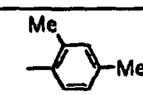 | Cu |

Table-1(3)

| Comp. | $R^1$ | $R^2$ | $R^3$ | $R^4$ | X | $R^6$ | $R^7$ | $R^8$ | $R^9$ | $R^{10}$ | $R^{11}$ | $R^{12}$ | M |
|---|---|---|---|---|---|---|---|---|---|---|---|---|---|
| 1−17 | H | H | H | H | S | Cl | H | H | Cl | H | H | 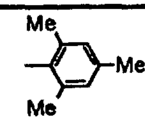 | Fe |
| 1−18 | H | H | $OCH_3$ | H | S | H | H | Br | H | H | H | 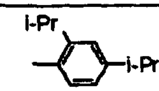 | Zn |
| 1−19 | H | H | H | H | $CH_2$ | I | H | H | Br | $CH_3$ | H | 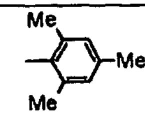 | Zn |
| 1−20 | H | H | H | H | $CHCH_3$ | H | H | H | H | Br | H | 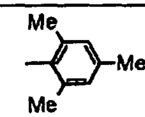 | Cu |
| 1−21 | $CH_3$ | H | H | Br | $CH_2$ | $CH_3$ | H | H | Br | Br | H | 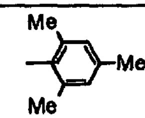 | Cu |
| 1−22 | Br | H | H | $CH_3$ | $CH_2$ | $CH_3$ | H | H | H | Br | H | 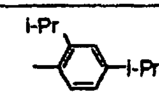 | Cu |
| 1−23 | H | Br | H | H | $CH_2$ | $C_2H_5$ | H | H | H | $CH_3$ | H | 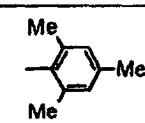 | Cu |
| 1−24 | H | Cl | H | H | $CH_2$ | $C_2H_5$ | H | H | H | $CH_3$ | H | 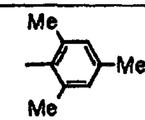 | Cu |

Table-1(4)

| Comp. | $R^1$ | $R^2$ | $R^3$ | $R^4$ | X | $R^6$ | $R^7$ | $R^8$ | $R^9$ | $R^{10}$ | $R^{11}$ | $R^{12}$ | M |
|---|---|---|---|---|---|---|---|---|---|---|---|---|---|
| 1-25 | H | H | H | H | S | Cl | H | H | Cl | H | H | 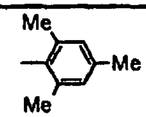 | Cu |
| 1-26 | H | H | $OCH_3$ | H | $CHOCH_3$ | H | H | Br | H | H | H | 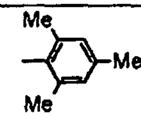 | Zn |
| 1-27 | H | H | H | H | $CH_2$ | I | H | H | Br | $CH_3$ | H | 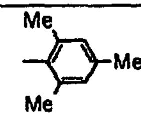 | Zn |
| 1-28 | H | H | H | Br | CHBr | H | H | H | H | $CH_3$ | H | 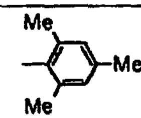 | Cu |
| 1-29 | H | $OCH_3$ | H | H | CHCl | $C_2H_5$ | H | H | H | $CH_3$ | H | 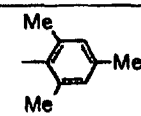 | Cu |
| 1-30 | H | $OCH_3$ | $OCH_3$ | H | $CH_2$ | $CH_3$ | H | H | H | $CH_3$ | H | 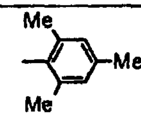 | Co |
| 1-31 | H | H | Br | H | $CH_2$ | $C_6H_{13}$ | H | H | Br | $CH_3$ | H | 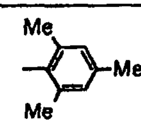 | Cu |
| 1-32 | H | H | Cl | H | $CH_2$ | $CH_3$ | H | H | Br | $CH_3$ | H | 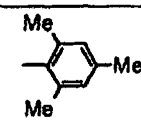 | Zn |

Table-1(5)

| Comp. | $R^1$ | $R^2$ | $R^3$ | $R^4$ | X | $R^6$ | $R^7$ | $R^8$ | $R^9$ | $R^{10}$ | $R^{11}$ | $R^{12}$ | M |
|---|---|---|---|---|---|---|---|---|---|---|---|---|---|
| 1-33 | H | H | H | H | $CH_2$ | $CF_3$ | H | H | H | $CH_3$ | H | 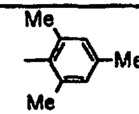 | Cu |
| 1-34 | H | H | H | H | $CH_2$ | $CH_2Ph$ | H | H | H | $CH_3$ | H | 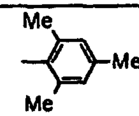 | Cu |
| 1-35 | H | H | H | H | $CH_2$ | $CH_2Ph$ | H | H | H | Br | H | 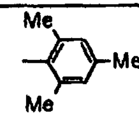 | Cu |
| 1-36 | H | H | H | H | $CH_2$ | $CH_2Ph$ | H | H | H | $CH_3$ | H | 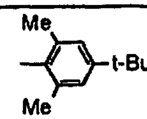 | Cu |
| 1-37 | H | H | H | H | $CH_2$ | $CH_2Ph$ | H | H | H | Br | H | 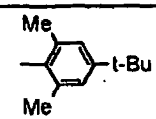 | Cu |
| 1-38 | H | H | H | H | O | $C_2H_5$ | H | H | H | $CH_3$ | H | 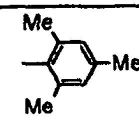 | Cu |
| 1-39 | H | H | H | H | O | $CH_3$ | H | H | H | $CH_3$ | H | 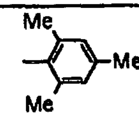 | Cu |
| 1-40 | H | H | H | H | O | $C_6H_{13}$ | H | H | Br | $CH_3$ | H | 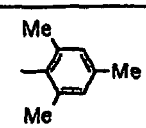 | Zn |

Table -1(6)

| Comp. | $R^1$ | $R^2$ | $R^3$ | $R^4$ | X | $R^6$ | $R^7$ | $R^8$ | $R^9$ | $R^{10}$ | $R^{11}$ | $R^{12}$ | M |
|---|---|---|---|---|---|---|---|---|---|---|---|---|---|
| 1−41 | H | H | H | H | O | $CH_3$ | H | H | Br | $CH_3$ | H | 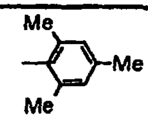 | Co |
| 1−42 | H | H | H | H | CHPh | $CH_3$ | H | H | H | Br | H | 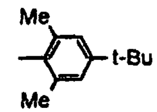 | Cu |
| 1−43 | H | H | H | H | CHPh | $C_2H_5$ | H | H | H | Br | H | 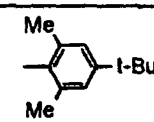 | Cu |
| 1−44 | H | H | H | H | CHPh | $CH_2Ph$ | H | H | H | $CH_3$ | H | 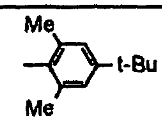 | Cu |
| 1−45 | H | H | H | H | CHPh | $CH_2Ph$ | H | H | H | $CH_3$ | H | 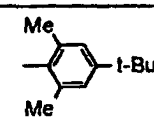 | Cu |
| 1−46 | H | H | H | H | CHPh | $C_2H_5$ | $CH_3$ | H | H | $CH_3$ | H | 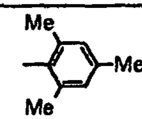 | Co |
| 1−47 | H | H | H | H | O | $CH_3$ | H | H | H | $CH_3$ | H | 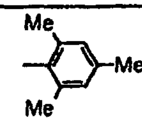 | Cu |
| 1−48 | H | H | H | H | O | $C_6H_{13}$ | H | H | Br | $CH_3$ | H | 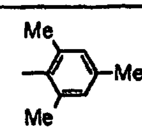 | Zn |

Table -1(7)

| Comp. | $R^1$ | $R^2$ | $R^3$ | $R^4$ | X | $R^6$ | $R^7$ | $R^8$ | $R^9$ | $R^{10}$ | $R^{11}$ | $R^{12}$ | M |
|---|---|---|---|---|---|---|---|---|---|---|---|---|---|
| 1−49 | H | H | H | H | O | $CH_3$ | H | H | Br | $CH_3$ | H | 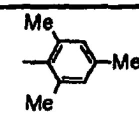 | Co |
| 1-50 | H | H | H | H | $CH_2$ | H | H | $CH_3$ | H | $CH_3$ | H | 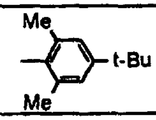 | Cu |
| 1-51 | H | H | H | H | $CH_2$ | $C_2H_5$ | H | $CH_3$ | H | $CH_3$ | H | 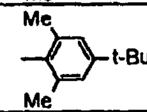 | Cu |
| 1-52 | H | H | H | H | $CH_2$ | Br | H | $CH_3$ | H | $CH_3$ | H | 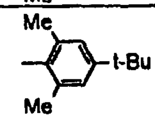 | Cu |
| 1-53 | H | H | H | H | $CH_2$ | H | H | H | $CH_3$ | Br | H | 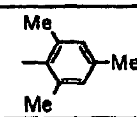 | Cu |
| 1-54 | H | H | H | H | $CH_2$ | $C_2H_5$ | H | H | $CH_3$ | Br | H | 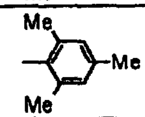 | Cu |
| 1-55 | H | H | H | H | $CH_2$ | Br | H | H | $CH_3$ | Br | H | 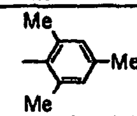 | Cu |
| 1-56 | H | H | H | H | S | H | H | $CH_3$ | H | $CH_3$ | H | 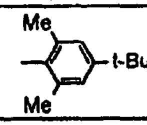 | Cu |
| 1-57 | H | H | H | H | S | $C_2H_5$ | H | $CH_3$ | H | $CH_3$ | H | 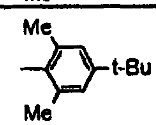 | Cu |

Table -1(8)

| Comp. | $R^1$ | $R^2$ | $R^3$ | $R^4$ | X | $R^6$ | $R^7$ | $R^8$ | $R^9$ | $R^{10}$ | $R^{11}$ | $R^{12}$ | M |
|---|---|---|---|---|---|---|---|---|---|---|---|---|---|
| 1-58 | H | H | H | H | S | Br | H | $CH_3$ | H | $CH_3$ | H | 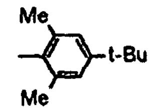 | Cu |
| 1-59 | H | H | H | H | S | H | H | H | $CH_3$ | Br | H | 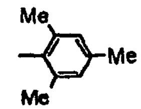 | Cu |
| 1−60 | H | H | H | H | S | $C_2H_5$ | H | H | $CH_3$ | Br | H | 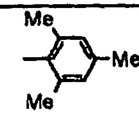 | Cu |
| 1−61 | H | H | H | H | S | Br | H | H | $CH_3$ | Br | H | 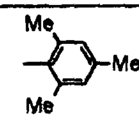 | Cu |
| 1−62 | H | H | H | H | O | H | H | $CH_3$ | H | $CH_3$ | H | 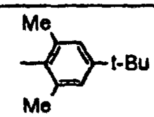 | Cu |
| 1−63 | H | H | H | H | O | $C_2H_5$ | H | $CH_3$ | H | $CH_3$ | H | 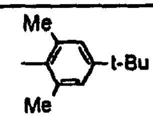 | Cu |
| 1−64 | H | H | H | H | O | Br | H | $CH_3$ | H | $CH_3$ | H | 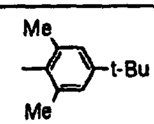 | Cu |
| 1−65 | H | H | H | H | O | H | H | H | $CH_3$ | Br | H | 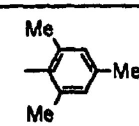 | Cu |

Table -1(9)

| Comp. | $R^1$ | $R^2$ | $R^3$ | $R^4$ | X | $R^6$ | $R^7$ | $R^8$ | $R^9$ | $R^{10}$ | $R^{11}$ | $R^{12}$ | M |
|---|---|---|---|---|---|---|---|---|---|---|---|---|---|
| 1−66 | H | H | H | H | O | $C_2H_5$ | H | H | $CH_3$ | Br | H | 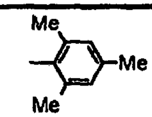 | Cu |
| 1−67 | H | H | H | H | O | Br | H | H | $CH_3$ | Br | H | 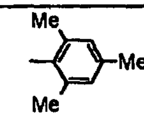 | Cu |
| 1−68 | H | H | H | (a−1) | $CH_2$ | $C_2H_5$ | H | H | H | Br | H | 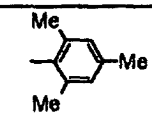 | Cu |
| 1−69 | H | H | H | H | $CH_2$ | (a−1) | H | H | H | $CH_3$ | H | 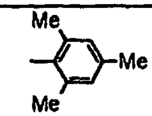 | Zn |
| 1−70 | H | H | H | H | $CH_2$ | H | H | H | (a−2) | H | H | 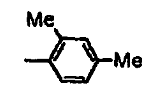 | Cu |
| 1−71 | H | H | H | H | $CH_2$ | $C(CH_3)_3$ | H | H | H | (a−3) | H | 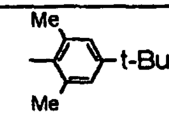 | Cu |
| 1−72 | H | H | H | H | $CH_2$ | $C(CH_3)_3$ | H | (a−4) | H | Br | H | 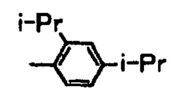 | Co |

Table-1(10)

| Comp. | $R^1$ | $R^2$ | $R^3$ | $R^4$ | X | $R^6$ | $R^7$ | $R^8$ | $R^9$ | $R^{10}$ | $R^{11}$ | $R^{12}$ | M |
|---|---|---|---|---|---|---|---|---|---|---|---|---|---|
| 1−73 | H | H | H | H | $CH_2$ | $CH_3$ | H | H | $CH_3$ | Br | (a−5) | 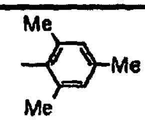 | Cu |
| 1−74 | H | H | H | H | $CH_2$ | $C(CH_3)_3$ | H | $CH_3$ | H | $CH_3$ | H | 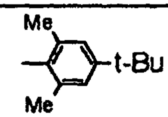 | Cu |
| 1−75 | H | H | H | H | S | $CH_2CH(CH_3)_2$ | H | H | $CH_3$ | Br | H | 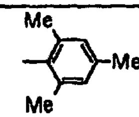 | Cu |
| 1−76 | H | H | H | H | S | $CH_2CH_2CH(CH_3)_2$ | H | H | $CH_3$ | Br | H | 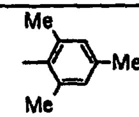 | Cu |
| 1−77 | H | H | H | H | $CH_2$ | $CH_2CH_2CH(CH_3)_2$ | H | H | H | Br | H | 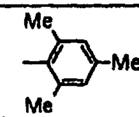 | Cu |
| 1−78 | H | H | H | H | O | $C(CH_3)_3$ | H | $CH_3$ | H | $CH_3$ | H | 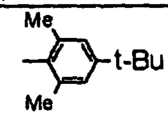 | Cu |
| 1−79 | H | H | H | H | O | $C(CH_3)_3$ | H | $CH_3$ | H | $CH_3$ | H | 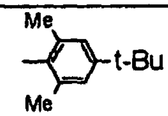 | Cu |
| 1−80 | H | H | H | H | CHPh | $C(CH_3)_3$ | H | $CH_3$ | H | $CH_3$ | H | 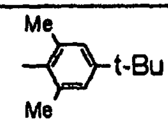 | Cu |

Table-2

$-CO-R^{13}$

| Substituent | $R^{13}$ |
|---|---|
| a-1 | $CH_3$ |
| a-2 | $C_6H_{11}$ |
| a-3 | $CH_2Ph$ |
| a-4 | 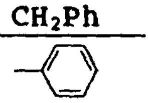 |
| a-5 | $C_2H_5$ |

For the mixture of dipyrromethene-metal chelate compounds represented by general formulas (3), (4) and (5), $R^{26}$ to $R^{29}$ and $R^{31}$ to $R^{32}$ each independently represent hydrogen, halogen, optionally substituted alkyl, alkoxy, aryl, aryloxy or a substituent represented by formula (b); $R^{33}$ represents halogen, optionally substituted alkyl, alkoxy, aryl or aryloxy; $R^{34}$ represents optionally substituted aryl; Z represents -O-, -S- or -CH($R^{30}$)-; $R^{30}$ represents hydrogen, halogen, optionally substituted alkyl, alkoxy, or aryl; and M'' represents transition element. Examples for each of these include those that were described above.

While the composition ratio of the respective constituents in the mixture of dipyrromethene-metal chelate compounds represented by general formulas (3), (4) and (5) is not particularly restricted, preferably the following conditions a), b), and c) are all satisfied.

a) The composition ratio of the dipyrromethene-metal chelate compound represented by general formula (3) is from 10% or more to less than 90%.
b) The composition ratio of the dipyrromethene-metal chelate compound represented by general formula (4) is from 10% or more to less than 90%.
c) The composition ratio of the dipyrromethene-metal chelate compound represented by general formula (5) is from 10% or more to less than 90%.

The mixture of dipyrromethene-metal chelate compounds represented by general formulas (3), (4) and (5) may be obtained, although not particularly restricted, by reacting in an appropriate solvent a mixture of a dipyrromethene compound represented by general formula (15) and a dipyrromethene compound represented by general formula (16) with the acetate or halide of a metal such as nickel, cobalt, iron, ruthenium, rhodium, palladium, copper, osmium, iridium, platinum, and zinc.

The mass ratio that the dipyrromethene compound represented by general formula (15) accounts for of the total mass of the dipyrromethene compound represented by general formula (15) and the dipyrromethene compound represented by general formula (16) is preferably from 10% or more to less than 90%, more preferably from 20% or more to less than 80%.

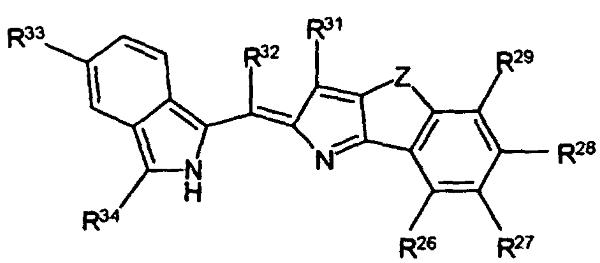

(15)

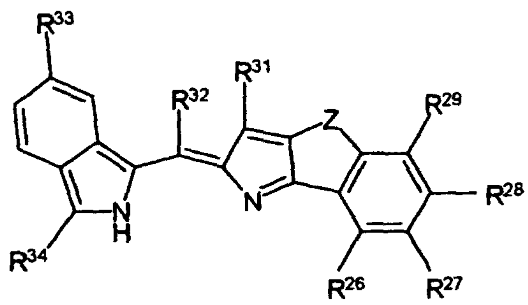

(16)

wherein in formulas (15) and (16), $R^{26}$ to $R^{34}$ and Z have the same meaning as that described above.

The dipyrromethene compound represented by general formula (15) or general formula (16) may be, for example, prepared as described in, but not limited to, Aust. J. Chem, 1965, 11, 1835-45, Heteroatom Chemistry, Vol. 1, 5,389(1990), U.S. Pat. No. 4,774,339 or U.S. Pat. No. 5,433,896.

That is, a compound represented by general formula (17) is reacted with a compound represented by general formula (18), or a compound represented by general formula (19) is reacted with a compound represented by general formula (20) in the presence of an acid catalyst such as hydrobromic acid and hydrochloric acid in an appropriate solvent, to obtain a dipyrromethene compound represented by general formula (15).

In the same manner, a compound represented by general formula (21) is reacted with a compound represented by general formula (18), or a compound represented by general formula (22) is reacted with a compound represented by general formula (20), in the presence of an acid catalyst such as hydrobromic acid and hydrochloric acid in an appropriate solvent, to obtain a dipyrromethene compound represented by general formula (16).

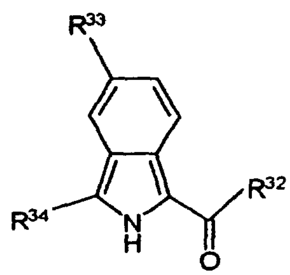

(17)

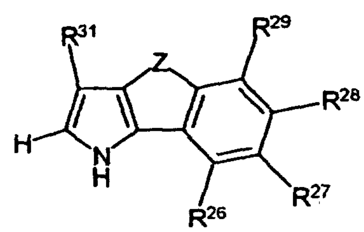

(18)

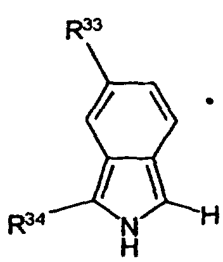

(19)

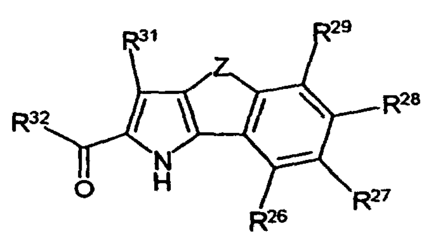

(20)

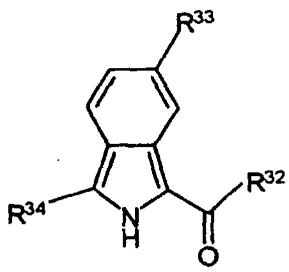

(21)

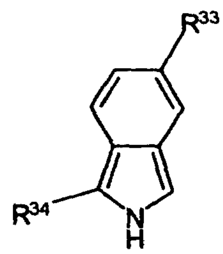

(22)

wherein in formulas (17) to (22), $R^{26}$ to $R^{34}$ and Z have the same meaning as that described above.

Although the mixture of the dipyrromethene compound represented by general formula (15) and the dipyrromethene compound represented by general formula (16) can be obtained by mixing both the dipyrromethene compound represented by general formula (15) and the dipyrromethene compound represented by general formula (16) that are prepared as described above, the mixture may also be obtained by copreparation in the following manner.

That is, a mixture of the compound represented by general formula (17) and the compound represented by general formula (21), and the compound represented by general formula (18), or a mixture of the compound represented by general formula (19) and the compound represented by general formula (22), and the compound represented by general formula (20), is reacted in the presence of an acid catalyst such as hydrobromic acid and hydrochloric acid in an appropriate solvent, to obtain a mixture of the dipyrromethene compounds represented by general formula (15) and general formula (16).

Specific examples of the dipyrromethene-metal chelate compound mixture represented by general formulas (3), (4) and (5) according to the present invention are shown in Table-3.

Table-3(1)

| Mixture | Comp. (3) | Comp. (4) | Comp. (5) | $R^{26}$ | $R^{27}$ | $R^{28}$ | $R^{29}$ | Z | $R^{31}$ | $R^{32}$ | $R^{33}$ | $R^{34}$ | M" |
|---|---|---|---|---|---|---|---|---|---|---|---|---|---|
| m-1 | 3-1 | 4-1 | 5-1 | H | H | H | H | $CH_2$ | $C_2H_5$ | H | $CH_3$ | 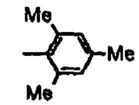 | Cu |
| m-2 | 3-2 | 4-2 | 5-2 | H | H | H | H | $CH_2$ | $C_2H_5$ | H | $CH_3$ | 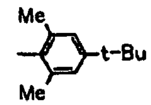 | Cu |
| m-3 | 3-3 | 4-3 | 5-3 | H | H | H | H | $CH_2$ | $CH_3$ | H | Br | 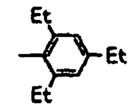 | Cu |
| m-4 | 3-4 | 4-4 | 5-4 | H | H | H | H | $CH_2$ | $CH_3$ | H | $CH_3$ | 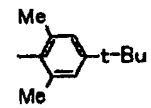 | Cu |
| m-5 | 3-5 | 4-5 | 5-5 | H | H | H | H | $CH_2$ | $C_2H_5$ | H | Br | 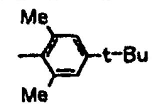 | Cu |
| m-6 | 3-6 | 4-6 | 5-6 | H | H | H | H | $CH_2$ | $CH_3$ | H | Br | 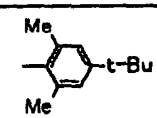 | Cu |
| m-7 | 3-7 | 4-7 | 5-7 | H | H | H | H | $CHCH_3$ | $CH_3$ | H | $CH_3$ | 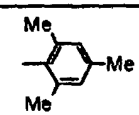 | Cu |
| m-8 | 3-8 | 4-8 | 5-8 | H | H | H | H | $CH_2$ | I | H | $CH_3$ | 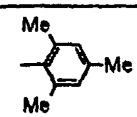 | Zn |

Table-3(2)

| Mixture | Comp. (3) | Comp. (4) | Comp. (5) | $R^{26}$ | $R^{27}$ | $R^{28}$ | $R^{29}$ | Z | $R^{31}$ | $R^{32}$ | $R^{33}$ | $R^{34}$ | M" |
|---|---|---|---|---|---|---|---|---|---|---|---|---|---|
| m-9 | 3-9 | 4-9 | 5-9 | H | H | H | H | $CHCH_3$ | H | H | Br | 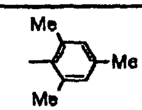 | Cu |
| m-10 | 3-10 | 4-10 | 5-10 | $CH_3$ | H | H | Br | $CH_2$ | $CH_3$ | H | Br | 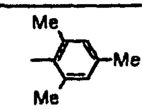 | Cu |
| m-11 | 3-11 | 4-11 | 5-11 | Br | H | H | $CH_3$ | $CH_2$ | $CH_3$ | H | Br | 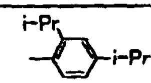 | Cu |
| m-12 | 3-12 | 4-12 | 5-12 | H | Br | H | H | $CH_2$ | $C_2H_5$ | H | $CH_3$ | 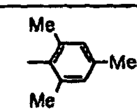 | Cu |
| m-13 | 3-13 | 4-13 | 5-13 | H | Cl | H | H | $CH_2$ | $C_2H_5$ | H | $CH_3$ | 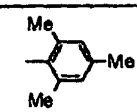 | Cu |
| m-14 | 3-14 | 4-14 | 5-14 | H | H | H | H | $CH_2$ | I | H | $CH_3$ | 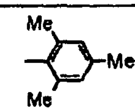 | Zn |
| m-15 | 3-15 | 4-15 | 5-15 | H | H | H | Br | CHBr | H | H | $CH_3$ | 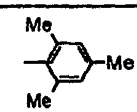 | Cu |
| m-16 | 3-16 | 4-16 | 5-16 | H | $OCH_3$ | H | H | CHCl | $C_2H_5$ | H | $CH_3$ | 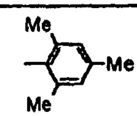 | Cu |

Table-3(3)

| Mixture | Comp. (3) | Comp. (4) | Comp. (5) | $R^{26}$ | $R^{27}$ | $R^{28}$ | $R^{29}$ | Z | $R^{31}$ | $R^{32}$ | $R^{33}$ | $R^{34}$ | M" |
|---|---|---|---|---|---|---|---|---|---|---|---|---|---|
| m-17 | 3-17 | 4-17 | 5-17 | H | $OCH_3$ | $OCH_3$ | H | $CH_2$ | $CH_3$ | H | $CH_3$ | 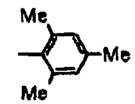 | Co |
| m-18 | 3-18 | 4-18 | 5-18 | H | H | Br | H | $CH_2$ | $C_6H_{13}$ | H | $CH_3$ | 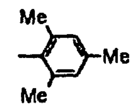 | Cu |
| m-19 | 3-19 | 4-19 | 5-19 | H | H | Cl | H | $CH_2$ | $CH_3$ | H | $CH_3$ | 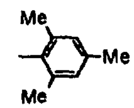 | Zn |
| m-20 | 3-20 | 4-20 | 5-20 | H | H | H | H | $CH_2$ | $CF_3$ | H | $CH_3$ | 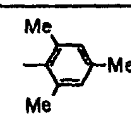 | Cu |
| m-21 | 3-21 | 4-21 | 5-21 | H | H | H | H | $CH_2$ | $CH_2Ph$ | H | $CH_3$ | 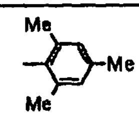 | Cu |
| m-22 | 3-22 | 4-22 | 5-22 | H | H | H | H | $CH_2$ | $CH_2Ph$ | H | Br | 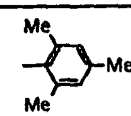 | Cu |
| m-23 | 3-23 | 4-23 | 5-23 | H | H | H | H | $CH_2$ | $CH_2Ph$ | H | $CH_3$ | 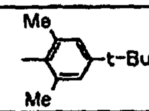 | Cu |
| m-24 | 3-24 | 4-24 | 5-24 | H | H | H | H | $CH_2$ | $CH_2Ph$ | H | Br | 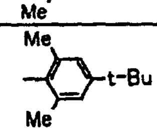 | Cu |

Table-3(4)

| Mixture | Comp. (3) | Comp. (4) | Comp. (5) | $R^{26}$ | $R^{27}$ | $R^{28}$ | $R^{29}$ | Z | $R^{31}$ | $R^{32}$ | $R^{33}$ | $R^{34}$ | M" |
|---|---|---|---|---|---|---|---|---|---|---|---|---|---|
| m-25 | 3-25 | 4-25 | 5-25 | H | H | H | H | O | $C_2H_5$ | H | $CH_3$ | 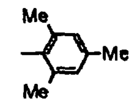 | Cu |
| m-26 | 3-26 | 4-26 | 5-26 | H | H | H | H | O | $CH_3$ | H | $CH_3$ | 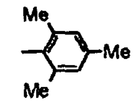 | Cu |
| m-27 | 3-27 | 4-27 | 5-27 | H | H | H | H | O | $C_6H_{13}$ | H | $CH_3$ | 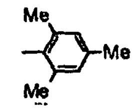 | Zn |
| m-28 | 3-28 | 4-28 | 5-28 | H | H | H | H | O | $CH_3$ | H | $CH_3$ | 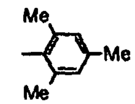 | Co |
| m-29 | 3-29 | 4-29 | 5-29 | H | H | H | H | CHPh | $CH_3$ | H | Br | 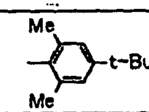 | Cu |
| m-30 | 3-30 | 4-30 | 5-30 | H | H | H | H | CHPh | $C_2H_5$ | H | Br | 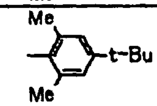 | Cu |
| m-31 | 3-31 | 4-31 | 5-31 | H | H | H | H | CHPh | $CH_2Ph$ | H | $CH_3$ | 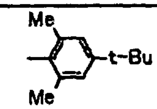 | Cu |
| m-32 | 3-32 | 4-32 | 5-32 | H | H | H | H | CHPh | $CH_2Ph$ | H | $CH_3$ | 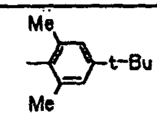 | Cu |

Table-3(5)

| Mixture | Comp. (3) | Comp. (4) | Comp. (5) | $R^{26}$ | $R^{27}$ | $R^{28}$ | $R^{29}$ | Z | $R^{31}$ | $R^{32}$ | $R^{33}$ | $R^{34}$ | M" |
|---|---|---|---|---|---|---|---|---|---|---|---|---|---|
| m-33 | 3-33 | 4-33 | 5-33 | H | H | H | H | CHPh | $C_2H_5$ | $CH_3$ | $CH_3$ | 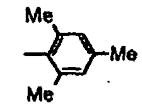 | Co |
| m-34 | 3-34 | 4-34 | 5-34 | H | H | H | H | O | $CH_3$ | H | $CH_3$ | 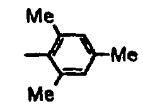 | Cu |
| m-35 | 3-35 | 4-35 | 5-35 | H | H | H | H | O | $C_6H_{13}$ | H | $CH_3$ | 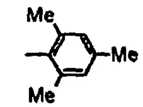 | Zn |
| m-36 | 3-36 | 4-36 | 5-36 | H | H | H | H | O | $CH_3$ | H | $CH_3$ | 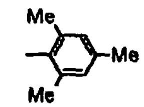 | Co |
| m-37 | 3-37 | 4-37 | 5-37 | H | H | H | H | $CH_2$ | H | H | $CH_3$ | 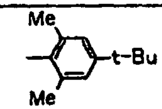 | Cu |
| m-38 | 3-38 | 4-38 | 5-38 | H | H | H | H | $CH_2$ | $C_2H_5$ | H | $CH_3$ | 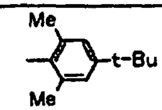 | Cu |
| m-39 | 3-39 | 4-39 | 5-39 | H | H | H | H | $CH_2$ | Br | H | $CH_3$ | 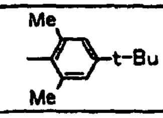 | Cu |
| m-40 | 3-40 | 4-40 | 5-40 | H | H | H | H | $CH_2$ | H | H | Br | 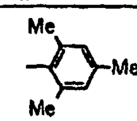 | Cu |

Table-3(6)

| Mixture | Comp. (3) | Comp. (4) | Comp. (5) | $R^{26}$ | $R^{27}$ | $R^{28}$ | $R^{29}$ | Z | $R^{31}$ | $R^{32}$ | $R^{33}$ | $R^{34}$ | M" |
|---|---|---|---|---|---|---|---|---|---|---|---|---|---|
| m-41 | 3-41 | 4-41 | 5-41 | H | H | H | H | $CH_2$ | $C_2H_5$ | H | Br | 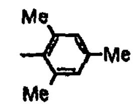 | Cu |
| m-42 | 3-42 | 4-42 | 5-42 | H | H | H | H | $CH_2$ | Br | H | Br | 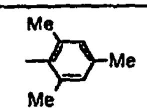 | Cu |
| m-43 | 3-43 | 4-43 | 5-43 | H | H | H | H | S | $C_2H_5$ | H | $CH_3$ | 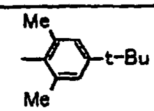 | Cu |
| m-44 | 3-44 | 4-44 | 5-44 | H | H | H | H | S | Br | H | $CH_3$ | 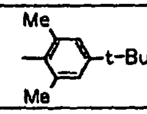 | Cu |
| m-45 | 3-45 | 4-45 | 5-45 | H | H | H | H | S | $C_2H_5$ | H | Br | 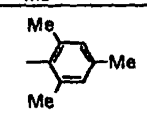 | Cu |
| m-46 | 3-46 | 4-46 | 5-46 | H | H | H | H | S | Br | H | Br | 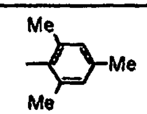 | Cu |
| m-47 | 3-47 | 4-47 | 5-47 | H | H | H | H | O | $C_2H_5$ | H | $CH_3$ | 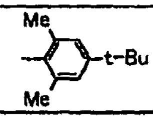 | Cu |
| m-48 | 3-48 | 4-48 | 5-48 | H | H | H | H | O | Br | H | $CH_3$ | 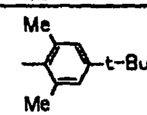 | Cu |

Table-3(7)

| Mixture | Comp. (3) | Comp. (4) | Comp. (5) | $R^{26}$ | $R^{27}$ | $R^{28}$ | $R^{29}$ | Z | $R^{31}$ | $R^{32}$ | $R^{33}$ | $R^{34}$ | M" |
|---|---|---|---|---|---|---|---|---|---|---|---|---|---|
| m-49 | 3-49 | 4-49 | 5-49 | H | H | H | H | O | $C_2H_5$ | H | Br | 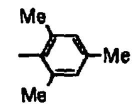 | Cu |
| m-50 | 3-50 | 4-50 | 5-50 | H | H | H | H | O | Br | H | Br | 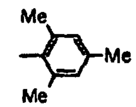 | Cu |
| m-51 | 3-51 | 4-51 | 5-51 | H | H | H | (a-1) | $CH_2$ | $C_2H_5$ | H | Br | 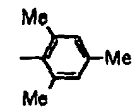 | Cu |
| m-52 | 3-52 | 4-52 | 5-52 | H | H | H | H | $CH_2$ | (a-1) | H | $CH_3$ | 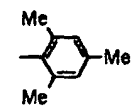 | Zn |
| m-53 | 3-53 | 4-53 | 5-53 | H | (a-2) | H | H | $CH_2$ | H | H | Cl | 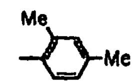 | Cu |
| m-54 | 3-54 | 4-54 | 5-54 | H | H | (a-3) | H | $CH_2$ | $C(CH_3)_3$ | H | I | 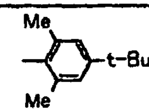 | Cu |
| m-55 | 3-55 | 4-55 | 5-55 | (a-4) | H | H | H | $CH_2$ | $C(CH_3)_3$ | H | Ph | 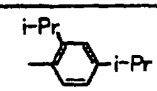 | Co |

Table-3(8)

| Mixture | Comp. (3) | Comp. (4) | Comp. (5) | $R^{26}$ | $R^{27}$ | $R^{28}$ | $R^{29}$ | Z | $R^{31}$ | $R^{32}$ | $R^{33}$ | $R^{34}$ | M" |
|---|---|---|---|---|---|---|---|---|---|---|---|---|---|
| m-56 | 3-56 | 4-56 | 5-56 | H | H | H | H | S | $CH_2CH(CH_3)_2$ | H | Br | 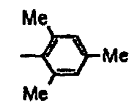 | Cu |
| m-57 | 3-57 | 4-57 | 5-57 | H | H | H | H | S | $CH_2CH_2\ CH(CH_3)_2$ | H | Br | 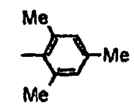 | Cu |
| m-58 | 3-58 | 4-58 | 5-58 | H | H | H | H | $CH_2$ | $CH_2CH_2\ CH(CH_3)_2$ | H | Br | 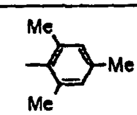 | Cu |
| m-59 | 3-59 | 4-59 | 5-59 | H | H | H | H | O | $C(CH_3)_3$ | H | $CH_3$ | 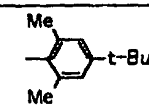 | Cu |
| m-60 | 3-60 | 4-60 | 5-60 | H | H | H | H | O | $C(CH_3)_3$ | H | OPh | 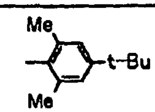 | Cu |
| m-61 | 3-61 | 4-61 | 5-61 | H | H | H | H | CHPh | $C(CH_3)_3$ | H | $OCH_3$ | 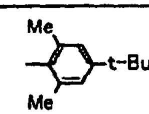 | Cu |

The structure of the recording medium according to the present invention will now be described.
The term optical recording medium denotes both an optical read-only medium that only regenerates prerecorded information, and an optical recording medium that can record information for regeneration. However, in this specification illustration will be made with regard to the latter optical recording medium which can record information for regeneration, and in particular, with regard to the optical recording medium which has a recording layer formed on the substrate and a reflective layer. The optical recording medium according to the present invention preferably has a laminate structure, as shown in Figure 1. That is to say, a recording layer 2 is formed on a substrate 1, and a reflective layer 3 is closely disposed thereon. On this reflective layer 3, another substrate 5 is further stuck thereon via an adhesive layer 4. However, another layer may be formed under or on the recording layer 2, or another layer may be formed on the reflective layer 3. Here, for reasons of compatibility with existing DVDs, the disc substrate is a 2 ply laminated structure having a thickness of 0.6 mm, outer diameter of 120 mmϕ and inner diameter of 15 mmϕ.
Next, the required characteristics of the structural respective layers for the optical recording medium according to the present invention and the structural materials thereof will be described.

1) Substrate

A material for the substrate should basically be transparent at wavelengths of recording light and regenerating light. For example, a polymeric material can be utilized such as polycarbonate resin, vinyl chloride resin, acrylic resin such as polymethyl methacrylate, polystyrene resin or an epoxy resin, or an inorganic material such as a glass. The substrate material is molded into disc substrates by injection molding or the like. Pre-grooves that express the recording position or pre-pits, some of which are reserved for read-only information, may also be formed on the surface of the substrate. Such pre-grooves or pre-pits are usually transferred at the time of the molding of the substrate by injection molding from a stamper template. They can also be manufactured by laser cutting (pre-write) or 2P (Photo Polymer) methods.
The substrate groove pitch is from 0.7 μm to 1.0 μm, and groove depth is from 100 nm to 200 nm, preferably 140 nm to 185 nm. Groove width is from 0.25 μm to 0.40 μm, preferably 0.30 μm to 0.35 μm. When groove depth is 100 nm or less, it is difficult to obtain the push-pull signal amplitude for tracking, and when 200 nm or more, the transfer process during injection molding is not practical production-wise. Furthermore, when groove depth is 0.25 μm or less, crosstalk becomes worse, and when 0.4 μm or more, the transfer process during injection molding is not practical production-wise. The shape of these grooves can be determined from profiles of AFM or optical diffraction analysis using He-Cd laser irradiation and the like.

2) Recording layer

The recording layer comprises a dipyrromethene metal chelate compound, of which the initial principal weight loss temperature is thermogravimetrically from 330°C through 500°C inclusive, and in particular comprises at least one dipyrromethene metal chelate compound represented by general formula (1).
Generally, one of the conditions that is thermally necessary for an organic dye used in a recording layer is that the initial principal weight loss temperature from thermogravimetric analysis is required to be within a certain temperature range. Specifically, it is considered that the temperature is preferably in the range of 200 to 350°C, because it was thought that if the initial weight loss temperature exceeds 350°C, the power of the laser beam becomes impracticably high, while if the temperature is below 200°C, recording stability deteriorates, such as causing regeneration degradation.
However, to carry out high-speed high-density recording without impairing recording sensitivity, and properly maintaining pit edges, it is important to stably form small pit sizes corresponding to high density. For this reason, it is required to form the pits in a short time using a highly focused, high-power laser beam, wherein it was found that for a dye within the conventional initial weight loss temperature range, a recording layer dye exposed to such conditions did not sufficiently achieve sharpening of the pit edges or right-sizing of the pit sizes due to the influence of rapid heat-generated decomposition upon a laser irradiation and the influence of residual heat after pit formation. Further, although the focused laser beam spot surroundings are effected by diffracted light, using a more powerful laser increases the effects of diffracted light.
However, in the present invention, attention was given to dipyrromethene metal complex dyes as a dye system that is excellent in light resistance and free from rapid heat-generated decomposition, wherein as a result of various investigations it was discovered that a strong correlation existed between initial principal weight loss temperature from thermogravimetric analysis and thermal interference degree during high speed recording. In particular, because in high speed recording the acquisition of recording sensitivity is important, it is required to set the extinction coefficient (k) at a high value, although there are concerns about the effects of the resulting thermal interference (residual heat). Under an advantageous film thickness condition to obtain degree of modulation (but the film thickness is not made to be

extremely thin), the change in the film due to thermal interference is also a problem. However, it was found that problems resulting from an increased temperature of the initial principal weight loss temperature were improved.

Accordingly, it was found that in the present invention, when a dipyrromethene metal chelate compound is selected as the recording layer dye, and the initial principal weight loss temperature thereof is from 330°C or more to 500°C or less, or preferably in the range of 350°C to 450°C, deterioration in pit jitter from thermal interference during high speed recording can be sufficiently moderately suppressed. In particular, for the dipyrromethene metal chelate compound represented by general formula (1), a compound can be achieved that has high light resistance and is within the above-described range for initial principal weight loss temperature. Here, if the initial principal weight loss temperature exceeds 500°C, the required power for the recording laser is too high, which is not practical, and if the temperature is below 330°C, alleviation of the thermal interference cannot be sufficiently achieved, so that jitter performance during high speed recording deteriorates.

In addition, the pit size formed from heat generation by laser irradiation is correlates with the heat weight loss slope and total weight loss. In a system in which the heat weight loss slope exceeds 2%/°C and the total weight loss exceeds 50%, overall recording pit formation is rapid, which is not suitable for formation of a pit size more minute than the spot to be subjected to the recording laser beam. Effective in acquiring a minute pit size is where the heat weight loss slope is from 2%/°C or less to 0.05%/°C or more, preferably 1.5%/°C or less to 0.1%/°C or more. Moreover, under those heat weight loss slope conditions, a total weight loss of from 15% or more to 75% or less, preferably 20% or more to 60% or less, is suitable.

Even regarding the calorific value resulting from dye decomposition due to laser irradiation, using a smaller amount of dye is preferable for stabilizing pit shape during high speed/high density recording. The decomposition calorific value is not more than 0.3 kJ/g, preferably not more than 0.2 kJ/g, as measured by DSC differential thermogravimetric analysis under nitrogen atmosphere.

In the present invention, the initial thermal weight loss temperature and weight loss slope is illustrated in Figure 2, and was determined within the following outline.

For example, Figure 2 illustrates the curve TG when an organic dye of mass $M_0$ was increasing by 10°C per minute in nitrogen. As a result of the increasing temperature, the mass at first decreased by a tiny amount, giving a generally straight line a-b weight loss line, and then began to suddenly decrease, decreasing at a weight loss of 15% or more along the generally straight line d1-d2. This is the principal weight loss process, wherein the initial principal weight loss temperature is at temperature T1, which corresponds to the intersection of lines a-b and d1-d2, and wherein the weight loss% at that time is taken as m1. Thereafter it settled down to the weight loss process illustrated by the line c-c. If the temperature at the intersection of lines d1-d2 and c-c is taken as T2 and the weight loss% as m2, the weight loss slope mentioned here is the value illustrated by the following equation (1),

$$|m2\text{-}m1|(\%)/(T2\text{-}T1)\ (^\circ C) \qquad (1)$$

and the weight loss% with respect to total mass (total weight loss%) is illustrated by the following equation (2)

$$|m2\text{-}m1|(\%) \qquad (2)$$

The initial principal weight loss temperature may be determined from differential calculus of this TG curve using the above concept.

The refractive index n of the recording layer single-layer with respect to the regeneration wavelength region is from 2.0 to 3.0, inclusive thereof, and preferably in the range of from 2.2 to 3.0, inclusive thereof. If n is less than 2.0, it is difficult to obtain sufficient optical change, so that the recording degree of modulation is undesirably low. If n is greater than 3.0, wavelength dependency becomes too high, wherein errors occur even in the recording regeneration wavelength region, which is not preferable. The extinction coefficient k is from 0.05 to 0.30, inclusive thereof, and preferably in the range of from 0.08 to 0.20, inclusive thereof. If k is less than 0.05, recording sensitivity deteriorates. If k is greater than 0.3, this is not preferable as it is difficult to obtain a reflectance of 45% or more.

To improve optical properties, recording sensitivity and signal characteristics, other dyes may be mixed in the recording layer. Examples of other dyes include cyanine dyes, squarylium dyes, naphthoquinone dyes, anthraquinone dyes, porphyrin dyes, azaporphyrin dyes, tetrapiraporphyrazine dyes, indophenol dyes, pyrylium dyes, thiopyrylium dyes, azulenium dyes, triphenylmethane dyes, xanthene dyes, indathlene dyes, indigo dyes, thioindigo dyes, melocyanine dyes, thiazine dyes, acridine dyes, oxadine dyes, phthalocyanine dyes and naphthalocyanine dyes, which may be used alone or in combination of two or more. While the mixing proportion of these dyes is generally about 0.1 to 30% of the dipyrromethene-metal chelate compound represented by general formula (1), or mixture of dipyrromethene-metal chelate compounds represented by general formulas (3) to (5), it is preferable to carry out mixing so that the

initial principal weight loss temperature in the mixing system is within the above-described range.

If necessary, a quencher, a dye-decomposition accelerator, an ultraviolet absorber, an adhesive, an endothermic decomposable compound and the like may be mixed into the recording layer, or alternatively can be chemically bonded to the dipyrromethene-metal chelate compound represented by general formula (1) or mixture of the dipyrromethene-metal chelate compounds represented by general formulas (3) to (5).

Examples of a quencher include metal complexes of acetylacetonates; bisdithiols such as bisdithio-$\alpha$-diketones and bisphenyldithiols; thiocathecols, salicylaldehyde oximes and thiobisphenolates. Amines are also preferable.

Examples of a thermal decomposition accelerator include metal compounds such as metal antiknock agents, metallocene compounds and acetylacetonate-metal complexes.

Furthermore, if necessary, a binder, leveling agent or an antifoaming agent may be combined. Preferable examples of a binder include polyvinyl alcohol, polyvinylpyrrolidone, nitrocellulose, cellulose acetate, ketone resins, acrylic resins, polystyrene resins, urethane resins, polyvinyl butyral, polycarbonate and polyolefins.

When forming the recording layer on a substrate, a layer made of an inorganic compound or a polymer may be formed on the substrate for improving solvent resistance of the substrate, reflectance or recording sensitivity.

The recording layer may be formed by, for example, application methods such as spin coating, spraying, casting and dipping; sputtering; chemical vapor deposition and vacuum deposition, although preferably spin coating because of its convenience. When using an application method such as spin coating, a coating is employed which has dispersed or dissolved in an organic solvent the dipyrromethene-metal chelate compound represented by general formula (1) or a mixture of the dipyrromethene-metal chelate compounds represented by general formulas (3) to (5) to 1 to 40 wt %, preferably 3 to 30 wt %. The organic solvent is preferably selected from those which do not damage the substrate. Examples of such a solvent include alcoholic solvents such as methanol, ethanol, isopropyl alcohol, octafluoropentanol, allyl alcohol, methylcellosolve, ethylcellosolve and tetrafluoropropanol; aliphatic or alicyclic hydrocarbon solvents such as hexane, heptane, octane, decane, cyclohexane, methylcyclohexane, ethylcyclohexane and dimethylcyclohexane; aromatic hydrocarbon solvents such as toluene, xylenes and benzene; halogenated hydrocarbon solvents such as carbon tetrachloride, chloroform, tetrachloroethane and dibromoethane; ether solvents such as diethyl ether, dibutyl ether, diisopropyl ether and dioxane; ketone solvents such as acetone and 3-hydroxy-3-methyl-2-butanone; ester solvents such as ethyl acetate and methyl lactate and the like, which may be used alone or in combination of two or more.

The thickness of the recording layer is preferably in a range wherein the thickness above the substrate's guiding groove (Groove) is from 30 nm to 150 nm. The thickness of the recording layer between guiding grooves (Land) is preferably in the range of from 10 nm to 80 nm. If the thickness on Groove exceeds 150 nm, in some cases the shortest pits will collapse, which is undesirable. If the thickness on Groove is thinner than 30 nm, good recording sensitivity and recording degree of modulation cannot always be achieved. An extremely thin film thickness on Land is especially preferable. Film thickness control of the recording layer is possible by using the above-described organic solvents in a plurality of mixtures.

3) Reflecting layer

On the recording layer, a reflecting layer is formed with a thickness of preferably 50 nm to 300 nm. The reflecting layer may be made of a material exhibiting an adequately high reflectance at a regenerating light wavelength; for example, metals such as Au, Al, Ag, Cu, Ti, Cr, Ni, Pt, Ta, Cr and Pd may be used alone or as an alloy. Among these, Au, Al and Ag are suitable as a reflecting layer material because of their higher reflectance. Apart from these, the reflecting layer may comprise another metal or metalloid such as Mg, Se, Hf, V, Nb, Ru, W, Mn, Re, Fe, Co, Rh, Ir, Zn, Cd, Ga, In, Si, Ge, Te, Pb, Po, Sn and Bi. A material comprising Au as a main component is suitable because it may easily provide a reflecting layer with a higher reflectance. A main component used herein refers to a component contained in a content of 50% or more. It may be possible to alternately laminate lower refractive index films and higher refractive index films made of materials other than a metal to form a multilayer film used as a reflecting layer.

The reflecting layer may be formed by, for example, sputtering, ion plating, chemical vapor deposition or vacuum deposition. An intermediate layer or adhesion layer made of a known inorganic or organic material may be formed on the substrate or under the reflecting layer for improving reflectance, recording properties, adhesiveness and the like.

4) Protective layer

There are no restrictions on the material for a protective layer as long as it can protect the reflecting layer from external effects. Examples of an organic substance used include thermoplastic resins, thermosetting resins, electron-beam curing resins and ultraviolet curing resins. Examples of an inorganic material used include $SiO_2$, $Si_3N_4$, $MgF_2$ and $SnO_2$. A thermoplastic or thermosetting resin dissolved in an appropriate solvent may be applied and dried

to form a protective layer. An ultraviolet curing resin may be applied as it is or as a coating solution thereof in an appropriate solvent and cured by irradiation of ultraviolet rays to form a layer. Examples of an ultraviolet curing resin which may be used include acrylate resins such as urethane acrylate, epoxyacrylate and polyester acrylate. These materials may be used alone or in combination of two or more and may be also used not only as a monolayer film but also as a multilayer film.

The protective layer may be formed, as described for the recording layer, by, for example, an application method such as spin coating and casting; sputtering; and chemical vapor deposition, preferably spin coating.

A thickness of the protective layer is generally 0.1 μm to 100 μm, although in the present invention it is 3 μm to 30 μm, more preferably 5 μm to 20 μm.

On the above-mentioned protective layer, a label and the like can also be further printed. In addition, there may be employed a means of laminating a protective sheet or a substrate on the surface of the reflective layer, or another means of each reflective layer of two optical recording.media coming in contact with each other to fix two optical recording media. For the purpose of protecting the surface or preventing the deposition of dust or the like, an ultraviolet curing resin layer, an inorganic thin film or the like may be formed on the mirror surface of the substrate.

The optical recording medium according to the present invention performs recording and regeneration using a laser beam having a wavelength of preferably 520 nm to 690 nm. For recording and regeneration using a laser beam having a wavelength of less than 520 nm, it is difficult in some cases to obtain a reflectance of 45% or more, while for recording and regeneration using a laser beam having a wavelength of more than 690 nm, recording sensitivity and recording degree of modulation can become low, so that using a laser beam within the above-described range is preferable.

A laser with a wavelength of 520 nm to 690 nm herein is for example, but not limited to, a dye laser whose wavelength may be selected in a wide visible-light range, a helium-neon laser with a wavelength of 633 nm, a high-output semiconductor layer with a wavelength of about 680, 650 or 635 nm which has been recently developed and a harmonic-converted YAG laser with a wavelength of 532 nm. The present invention may achieve higher-density recording and regenerating at one wavelength or multiple wavelengths selected from these.

The present invention will be now described with reference to, but not limited to, Examples.

(Example 1) Preparation of a Dipyrromethene-metal Chelate Compound (Compound 1-1)

In 500 g of ethanol were dissolved 8.7 g of the compound represented by structural formula (12-a) and 7.0 g of the compound represented by structural formula (13-a). Hydrobromic acid (6.6 g, 47%) was dropped into the resulting solution, and the mixture was stirred at room temperature for 3 hours. After concentration under reduced pressure, the residue was extracted with 500 g of chloroform and washed with water. The extracted solution was separated, then the solvent was evaporated off to give 11.0 g of the compound represented by structural formula (14-a).

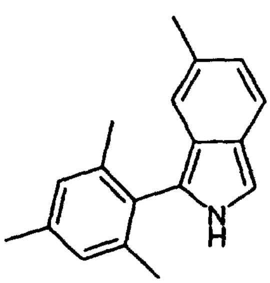

(12-a)

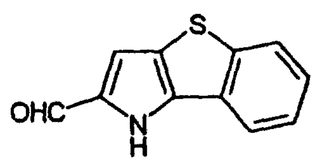

(13-a)

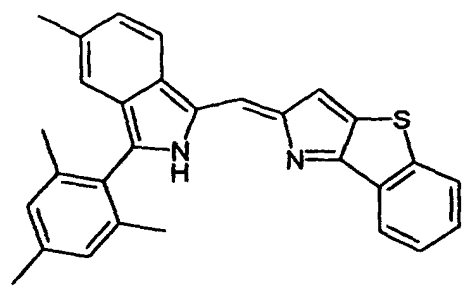

(14-a)

Then, in 200 g of ethanol and 200 g of toluene, 6.3 g of the compound represented by structural formula (14-a) was dissolved. After adding 1.52 g of copper acetate, the mixture was stirred at 50°C for 3 hours. After concentration under reduced pressure, the precipitate was collected by filtration and washed with methanol and water to give 3.0 g of the compound (1-1).

From the following analysis results, it was confirmed as the title compound.

| Elementary analysis: $C_{58}H_{46}N_4S_2Cu$ | | | |
|---|---|---|---|
| | C | H | N |
| Calculated(%) | 75.17 | 5.00 | 6.05 |
| Found(%) | 75.11 | 5.05 | 6.10 |
| MS (m/e): 925 ($M^+$) | | | |

The compound thus obtained exhibited in toluene a local absorption maximum at 576 nm and a gram absorption coefficient of 1.17 x $10^5$ ml/g.cm.

(Example 2) Preparation of a Dipyrromethene-metal Chelate Compound (Compound 1-4)

In 220 g of ethanol were dissolved 5.4 g of the compound represented by structural formula (12-b) and 3.2 g of the compound represented by structural formula (13-b). Hydrobromic acid (3.1 g, 47%) was dropped into the resulting solution, and the mixture was stirred at room temperature for 1 hour. After concentration under reduced pressure, the residue was extracted with 300 g of chloroform and washed with water. The extracted solution was separated, and then the solvent was evaporated off to give 5.5 g of the compound represented by structural formula (14-b).

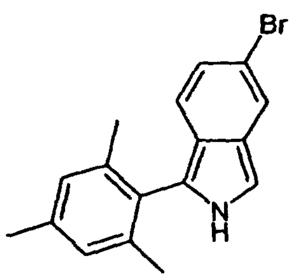

(12-b)

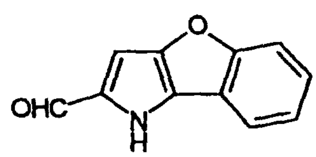

(13-b)

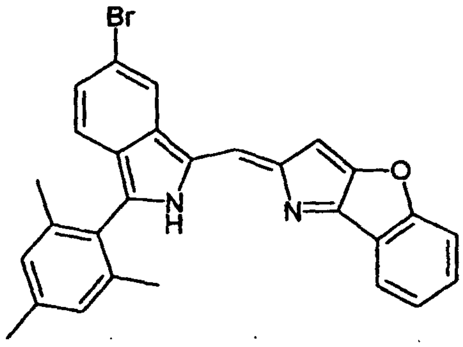

(14-b)

Then, in 200 g of ethanol and 100 g of toluene, 5.4 g of the compound represented by structural formula (14-b) was dissolved. After adding 1.30 g of copper acetate, the mixture was stirred at 50°C for 1 hour. After concentration under reduced pressure, the precipitate was collected by filtration and washed with methanol and water to give 5.0 g of the compound (1-4).

From the following analysis results, it was confirmed as the title compound.

| Elementary analysis: $C_{56}H_{40}N_4Br_2O_2Cu$ | | | |
|---|---|---|---|
| | C | H | N |
| Calculated (%) | 65.66 | 3.94 | 5.47 |
| Found (%) | 65.71 | 3.90 | 5.43 |
| MS (m/e): 1021 ($M^+$) | | | |

The compound thus obtained exhibited in toluene a local absorption maximum at 599 nm and a gram absorption coefficient of 1.31 x $10^5$ ml/g.cm.

(Example 3) Preparation of a Dipyrromethene-metal Chelate Compound (Compound 1-7)

In 72 g of ethanol were dissolved 1.8 g of the compound represented by structural formula (12-c) and 1.0 g of the compound represented by structural formula (13-c). Hydrobromic acid (0.92 g, 47%) was dropped into the resulting solution, and the mixture was stirred at room temperature for 1 hour. After concentration under reduced pressure, the residue was extracted with 100 ml of chloroform and washed with water. The solution was separated, and then the solvent was evaporated off to give 1.4 g of the compound represented by structural formula (14-c).

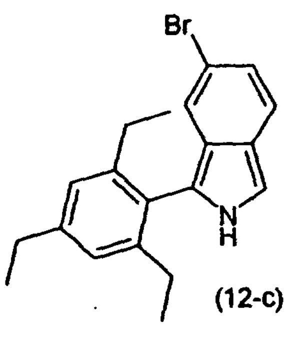

(12-c)

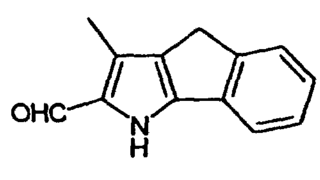

(13-c)

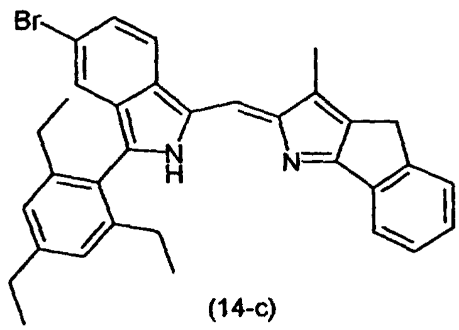

(14-c)

Then, in 56 g of ethanol and 25 g of toluene, 1.4 g of the compound represented by structural formula (14-c) was dissolved. After adding 0.3 g of copper acetate, the mixture was stirred at reflux temperature for 2 hours. After concentration under reduced pressure, the precipitate was collected by filtration and washed with methanol and water to give 1.0 g of the compound (1-7).

From the following analysis results, it was confirmed as the title compound.

| Elementary analysis: $C_{66}H_{60}N_4Br_2Cu$ | | | |
|---|---|---|---|
| | C | H | N |
| Calculated (%) | 69.99 | 5.34 | 4.95 |
| Found (%) | 70.01 | 5.49 | 4.93 |
| MS (m/e): 1129 ($M^+$) | | | |

The compound thus obtained exhibited in toluene a local absorption maximum at 599.5 nm and a gram absorption coefficient of 1.32 x $10^5$ ml/g.cm.

(Example 4) Preparation of a Dipyrromethene-metal Chelate Compound (Compound 1-9)

In 100 g of ethanol were dissolved 3.2 g of the compound represented by structural formula (12-d) and 1.9 g of the compound represented by structural formula (13-d). Hydrobromic acid (1.5 g, 47%) was dropped into the resulting solution, and the mixture was stirred at room temperature for 3 hours. After concentration under reduced pressure, the residue was extracted with 170 g of chloroform and washed with water. The solution was separated, and then the solvent was evaporated off to give 4.3 g of the compound represented by structural formula (14-d).

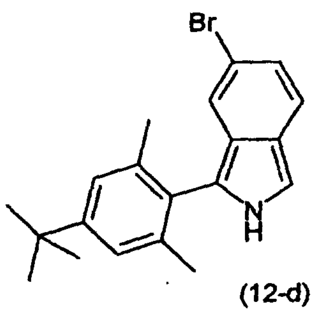

(12-d)

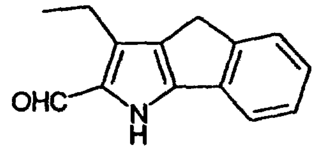

(13-d)

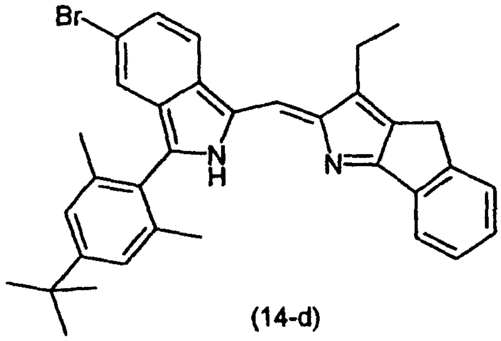

(14-d)

Then, in 50 g of ethanol and 50 g of toluene, 4.1 g of the compound represented by structural formula (14-d) was dissolved. After adding 0.75 g of copper acetate, the mixture was stirred at 50°C for 2 hours. After concentration under reduced pressure, the precipitate was collected by filtration and washed with methanol and water to give 4.3 g of the compound (1-9).

From the following analysis results, it was confirmed as the title compound.

| Elementary analysis: $C_{68}H_{65}N_4Br_2Cu$ | | | |
|---|---|---|---|
| | C | H | N |
| Calculated (%) | 70.37 | 5.56 | 4.83 |
| Found (%) | 70.33 | 5.55 | 4.81 |
| MS (m/e): 1157 ($M^+$) | | | |

The compound thus obtained exhibited in toluene a local absorption maximum at 600 nm and a gram absorption coefficient of 1.84 x $10^5$ ml/g.cm.

(Example 5) Preparation of a Dipyrromethene-metal Chelate Compound (Compound 1-11)

In 60 g of ethanol were dissolved 1.2 g of the compound represented by structural formula (12-a) and 1.0 g of the compound represented by structural formula (13-e). Hydrobromic acid (0.8 g, 47%) was dropped into the resulting solution, and the mixture was stirred at room temperature for 3 hours. After concentration under reduced pressure, the residue was extracted with 100 g of chloroform and washed with water. The solution was separated, and then the solvent was evaporated off to give 1.4 g of the compound represented by structural formula (14-e).

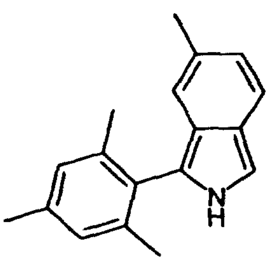

(12-a)

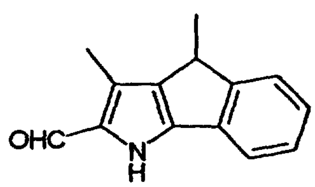

(13-e)

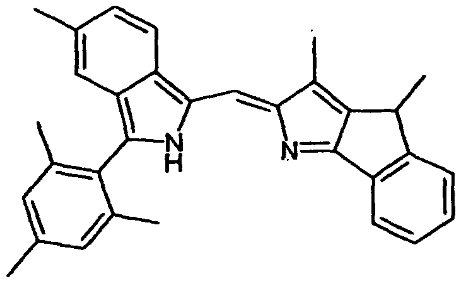

(14-e)

Then, in 60 g of ethanol and 20 g of toluene, 1.4 g of the compound represented by structural formula (14-e) was dissolved. After adding 0.34 g of copper acetate, the mixture was stirred at reflux temperature for 2 hours. After concentration under reduced pressure, the precipitate was collected by filtration and washed with methanol and water to give 1.4 g of the compound (1-11). From the following analysis results, it was confirmed as the title compound.

| Elementary analysis: $C_{64}H_{58}N_4Cu$ | | | |
|---|---|---|---|
| | C | H | N |
| Calculated (%) | 81.19 | 6.18 | 5.92 |
| Found (%) | 81.10 | 6.22 | 6.00 |
| MS (m/e): 945 ($M^+$) | | | |

The compound thus obtained exhibited in toluene a local absorption maximum at 601 nm and a gram absorption coefficient of 1.84 x $10^5$ ml/g.cm.

(Example 6) Preparation of a Dipyrromethene-metal Chelate Compound (Compound 1-15)

In 500 g of ethanol were dissolved 3.1 g of the compound represented by structural formula (12-e) and 1.8 g of the compound represented by structural formula (13-b). Hydrobromic acid (1.6 g, 47%) was dropped into the resulting solution, and the mixture was stirred at room temperature for 3 hours. After concentration under reduced pressure, the residue was extracted with 80 g of chloroform and washed with water. The solution was separated, and then the solvent was evaporated off to give 3.9 g of the compound represented by structural formula (14-f).

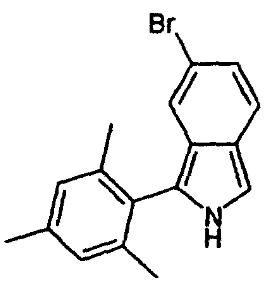

(12-e)

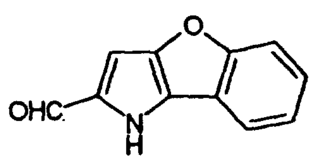

(13-b)

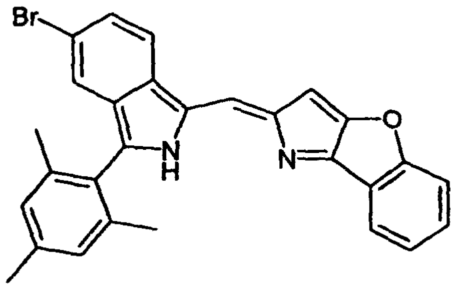

(14-f)

Then, in 60 g of ethanol and 20 g of toluene, 3.0 g of the compound represented by structural formula (14-f) was dissolved. After adding 0.8 g of cobalt acetate, the mixture was stirred at reflux temperature for 2 hours. After concentration under reduced pressure, the precipitate was collected by filtration and washed with methanol and water to give 2.5 g of the compound (1-15).

From the following analysis results, it was confirmed as the title compound.

| Elementary analysis: $C_{56}H_{40}N_4Br_2O_2Co$ | | | |
|---|---|---|---|
| | C | H | N |
| Calculated (%) | 65.96 | 3.95 | 5.49 |
| Found (%) | 66.05 | 3.85 | 5.35 |
| MS (m/e): 1017 ($M^+$) | | | |

The compound thus obtained exhibited in toluene a local absorption maximum at 625 nm and a gram absorption coefficient of 1.08 x $10^5$ ml/g.cm.

(Example 7) Preparation of a Dipyrromethene-metal Chelate Compound (Compound 1-42)

In 100 g of ethanol were dissolved 1.5 g of the compound represented by structural formula (12-d) and 1.0 g of the compound represented by structural formula (13-f). Hydrobromic acid (0.7 g, 47%) was dropped into the resulting solution, and the mixture was stirred at room temperature for 2 hours. After concentration under reduced pressure, the residue was extracted with 100 g of chloroform and washed with water. The solution was separated, and then the solvent was evaporated off to give 2.3 g of the compound represented by structural formula (14-g).

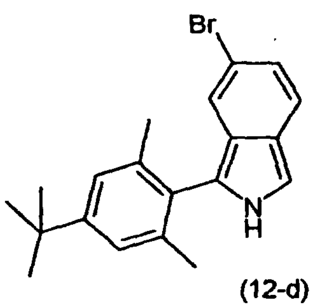

(12-d)

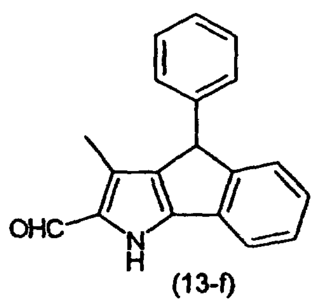

(13-f)

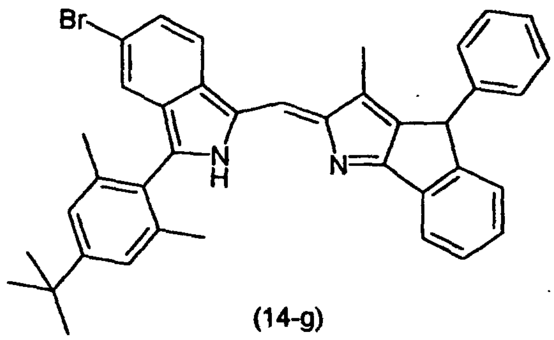

(14-g)

Then, in 60 g of ethanol and 20 g of toluene, 2.0 g of the compound represented by structural formula (14-g) was dissolved. After adding 0.4 g of copper acetate, the mixture was stirred at reflux temperature for 2 hours. After concentration under reduced pressure, the precipitate was collected by filtration and washed with methanol and water to give 1.5 g of the compound (1-42).

From the following analysis results, it was confirmed as the title compound.

| Elementary analysis: $C_{78}H_{68}N_4Br_2Cu$ | | | |
|---|---|---|---|
| | C | H | N |
| Calculated (%) | 72.92 | 5.33 | 4.36 |
| Found (%) | 72.85 | 5.22 | 4.30 |
| MS (m/e): 1281 ($M^+$) | | | |

The compound thus obtained exhibited in toluene a local absorption maximum at 599 nm and a gram absorption coefficient of $1.73 \times 10^5$ ml/g.cm.

(Examples 8-11)

A dipyrromethene-metal chelate compound listed in Table 1 was suitably dissolved, alone or as a mixture, into a mixed solvent containing ethylcyclohexane:xylene 10:1 (15 g/l) for depositing on an injection molded polycarbonate substrate having a thickness of 0.6 mm and a spiral groove with a diameter of 120 mmϕ (pitch: 0.74 µm, depth: 165 nm, width 0.33µm) by spin coating so that the film thickness above the groove was adjusted to about 80 nm and the film thickness between grooves to 20 nm. After the resulting layer was dried for 2 hours at 80°C, an AgPdCu reflecting film was formed thereon to a film thickness of 80 nm using a Balzers sputtering apparatus (CDI-900), then a UV-curing resin SD17 (manufactured by Dainippon Ink and Chemicals, Incorporated) was applied to this reflecting layer and subjected to UV curing. A 0.6 mm thickness polycarbonate substrate the same as that described above was laminated on top of this to prepare an optical recording medium laminated by UV light using a JSR manufactured KZ8681 radical polymerizing adhesive.

The optical constants at 650 nm (refractive index, extinction coefficient) and the initial thermal weight loss temperature under nitrogen atmosphere for this recording layer (measured using a TA-50WS Shimadzu TG Analyzer) are shown in Table 4.

A DVDR compatible EFM+ signal was recorded onto this optical recording medium using a Pulstec Industrial Co., Ltd. disc tester DDU1000 at wavelength 661 nm, NA=0.60; linear velocity 14 m/s (DVD-R normal recording speed for 4x-speed). The recording conditions were a high-speed drive of 4x-speed having pulse conditions as described in the DVD-R Specification (version 2.0), and recording was carried out by making optimal adjustment to each pit. These recorded sites were measured for jitter at a DVD standard speed.

Table 4 shows the results of the measured jitter at that time. For the DVDR medium illustrated in the present example, a satisfactory jitter value over a broad power window was confirmed.

(Comparative Examples 1 and 2)

An optical recording medium was prepared as described in Examples 8 to 11 using the dipyrromethene-metal chelate compound represented by the following structural formulas (A) and (B), and evaluated in the same manner. Both of the signal characteristics exceeded a jitter value of 10%, and satisfactory signal characteristics were not obtained.

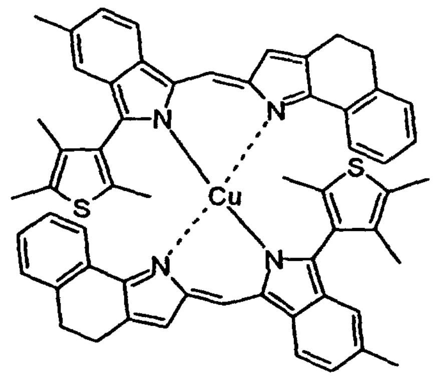

(A)

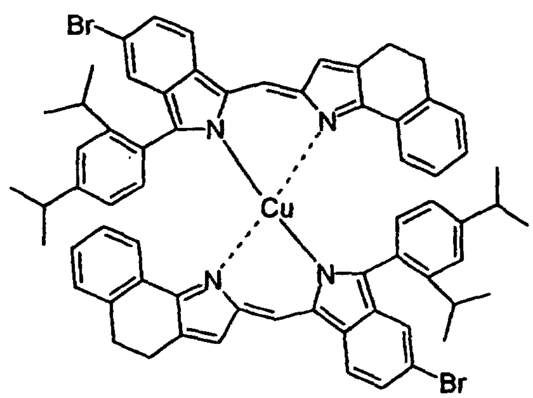

(B)

Table-4

| | Compound | Optical Properties | | TG Analysis | 4x-Speed Signal Characteristics | |
|---|---|---|---|---|---|---|
| | | Refractive Index n | Extinction Coefficient k | Initial Thermal Weight Loss Temperature °C | Jitter % | Degree of Modulation |
| Example 8 | 1-3 | 2.49 | 0.10 | 409 | 8.3 | 0.72 |
| Example 9 | 1-4 | 2.66 | 0.17 | 413 | 7.8 | 0.71 |
| Example 10 | 1-9 | 2.53 | 0.15 | 410 | 6.5 | 0.73 |
| Example 11 | 1-4 and formula (A) mixing ratio 1:1 | 2.67 | 0.12 | 408 | 8.4 | 0.70 |
| Comparative Example 1 | formula (A) | 2.36 | 0.12 | 290 | 11 | 0.65 |
| Comparative Example 2 | formula (B) | 2.67 | 0.17 | 280 | 14 | 0.62 |

(Example 12) Preparation of a Dipyrromethene-metal Chelate Compound Mixture (Compound Mixture m-5)

In 150 ml of ethanol were dissolved 3.00 g of the compound represented by structural formula (17-a) and 1.49 g of the compound represented by structural formula (18-a). Hydrobromic acid (1.40 g, 47%) was dropped into the resulting solution, and the mixture was stirred at room temperature for 2 hours. After concentration under reduced pressure, the residue was extracted with 200 ml of chloroform and washed with water. The solution was separated, then the solvent was evaporated off to give 4.02 g of the compound represented by structural formula (15-a).

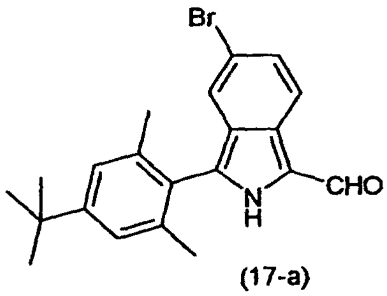

(17-a)

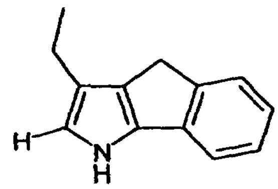

(18-a)

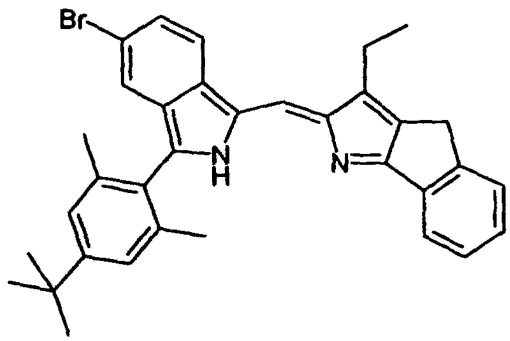

(15-a)

In the same manner, 6.00 g of the compound represented by structural formula (21-a) and 2.98 g of the compound represented by structural formula (18-a) was dissolved in 300 ml of ethanol. Hydrobromic acid (2.80 g, 47%) was dropped into the resulting solution, and the mixture was stirred at room temperature for 2 hours. After concentration under reduced pressure, the residue was extracted with 400 ml of chloroform and washed with water. The solution was separated, then the solvent was evaporated off to give 8.00 g of the compound represented by structural formula (16-a).

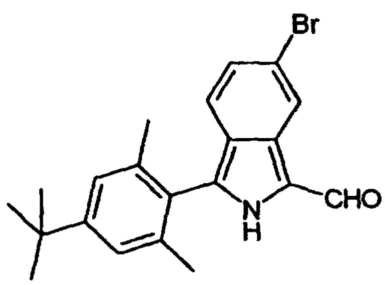

(21-a)

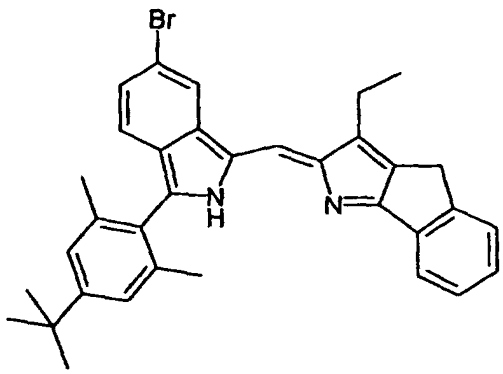

(16-a)

Then, in 400 g of ethanol, 4.00 g of the compound represented by structural formula (15-a) and 6.00 g of the compound represented by structural formula (16-a) were dissolved. After adding 3.30 g of copper acetate, the mixture was stirred at reflux temperature for 2 hours. After concentration under reduced pressure, the precipitate was collected by filtration and washed with methanol and water to give 8.98 g of the dipyrromethene-metal chelate compound mixture (m-5) represented by the structural formulas (3-5), (4-5) and (5-5).

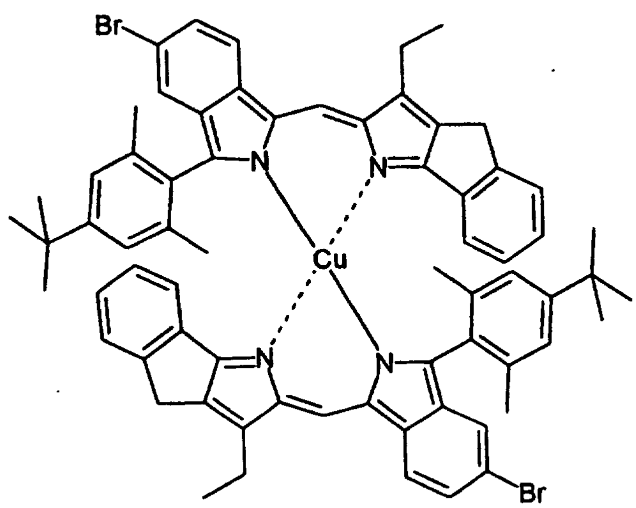

(3-5)

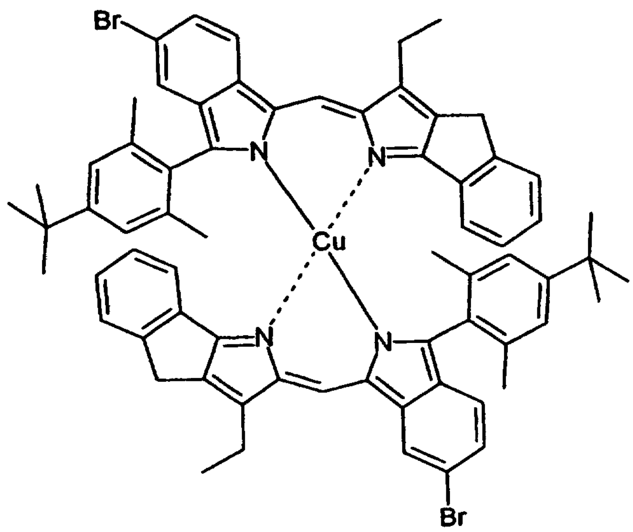

(4-5)

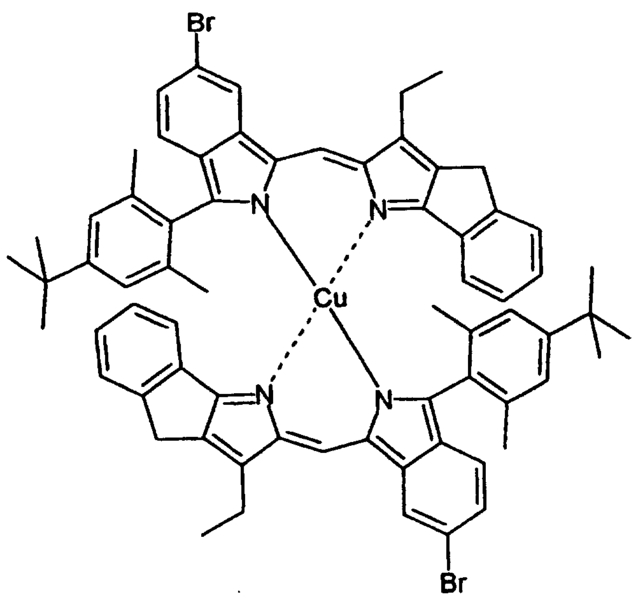

(5-5)

From the following analysis results, it was confirmed as the title compound.

| Elementary analysis: $C_{68}H_{64}N_4Br_2Cu$ | | | |
|---|---|---|---|
| | C | H | N |
| Calculated (%) | 70.37 | 5.56 | 4.83 |
| Found (%) | 70.22 | 5.50 | 4.88 |
| MS (m/e): 1157 ($M^+$) | | | |

In addition, from HPLC analysis it was determined that the respective compounds had the following composition ratio.

| Compound (3-5) | Compound (4-5) | Compound (5-5) |
|---|---|---|
| 33% | 47% | 20% |

The compounds thus obtained exhibited in toluene a local absorption maximum at 597.5 nm and a gram absorption coefficient of 1.77 x $10^5$ ml/g.cm.

(Example 13) Preparation of a Dipyrromethene-metal Chelate Compound Mixture (Compound Mixture m-41)

In 150 ml of ethanol were dissolved 3.00 g of the compound represented by structural formula (17-b) and 1.61 g of the compound represented by structural formula (18-a). Hydrobromic acid (1.55 g, 47%) was dropped into the resulting solution, and the mixture was stirred at room temperature for 2 hours. After concentration under reduced pressure, the residue was extracted with 200 ml of chloroform and washed with water. The solution was separated, and then the solvent was evaporated off to give 3.60 g of the compound represented by structural formula (15-b).

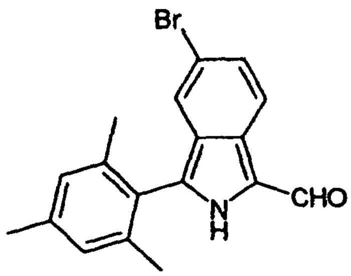

(17-b)

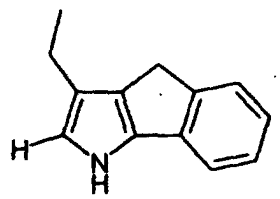

(18-a)

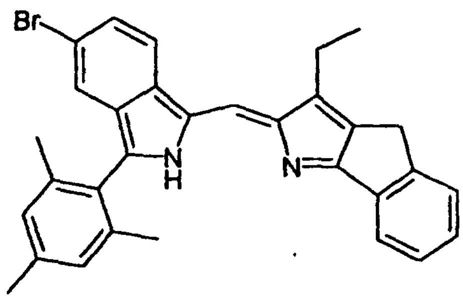

(15-b)

In the same manner, 6.00 g of the compound represented by structural formula (21-b) and 3.22 g of the compound represented by structural formula (18-a) was dissolved in 300 ml of ethanol. Hydrobromic acid (3.1 g, 47%) was dropped into the resulting solution, and the mixture was stirred at room temperature for 2 hours. After concentration under reduced pressure, the residue was extracted with 400 ml of chloroform and washed with water. The solution was separated, then the solvent was evaporated off to give 7.48 g of the compound represented by structural formula (16-b).

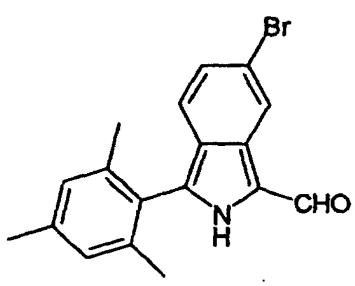

(21-b)

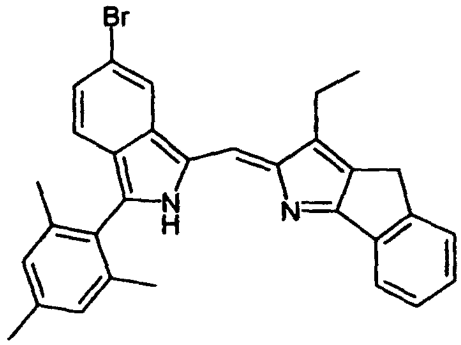

(16-b)

Then, in 400 ml of ethanol, 4.00 g of the compound represented by structural formula (15-b) and 6.00 g of the compound represented by structural formula (16-b) were dissolved. After adding 3.58 g of copper acetate, the mixture was stirred at reflux temperature for 2 hours. After concentration under reduced pressure, the precipitate was collected by filtration and washed with methanol and water to give 9.76 g of the dipyrromethene-metal chelate compound mixture (m-5) represented by the structural formulas (3-41), (4-41) and (5-41).

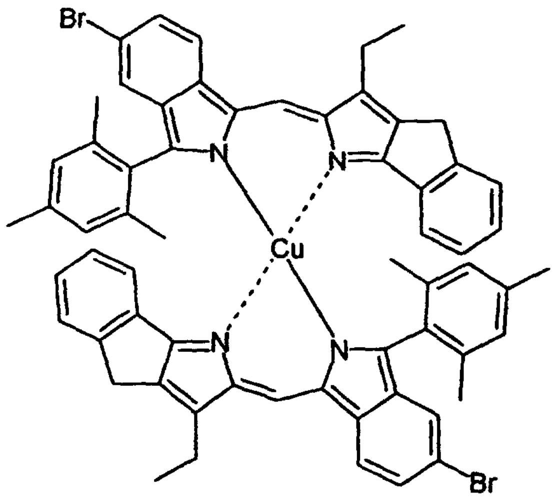

(3-41)

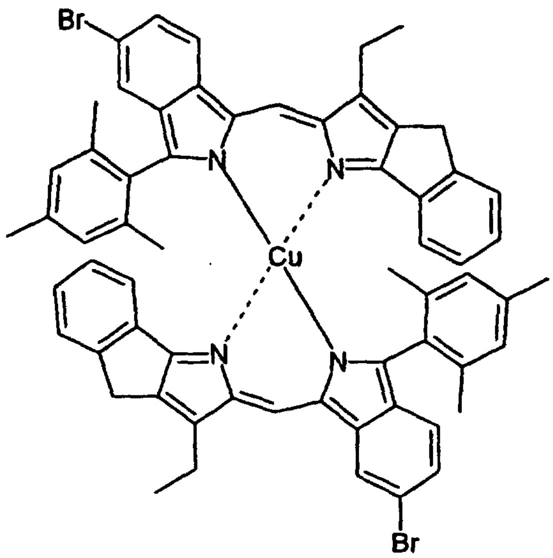

(4-41)

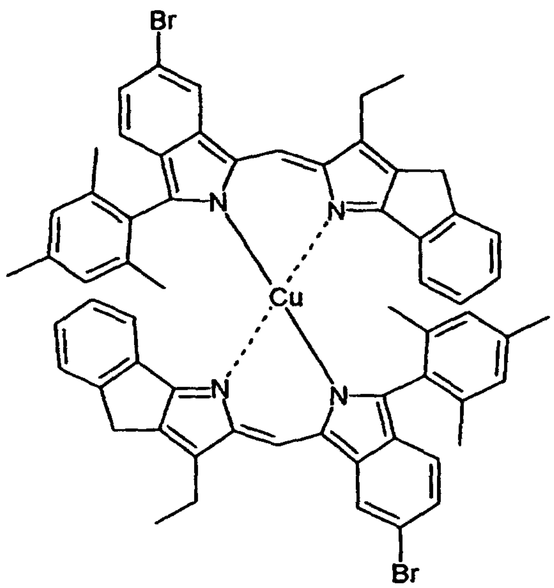

(5-41)

From the following analysis results, it was confirmed as the title compound.

| Elementary analysis: $C_{62}H_{52}N_4Br_2Cu$ | | | |
|---|---|---|---|
| | C | H | N |
| Calculated (%) | 69.18 | 4.87 | 5.20 |
| Found (%) | 68.99 | 4.90 | 5.22 |
| MS (m/e): 1073 ($M^+$) | | | |

In addition, from HPLC analysis it was determined that the respective compounds had the following composition ratio.

| (Compound 3-41) | Compound (4-41) | Compound (5-41) |
|---|---|---|
| 30% | 48% | 22% |

The compounds thus obtained exhibited in toluene a local absorption maximum at 599.0 nm and a gram absorption coefficient of 1.73 x $10^5$ ml/g.cm.

(Example 14) Preparation of a Dipyrromethene-metal Chelate Compound Mixture (Compound Mixture m-43)

In 150 ml of ethanol were dissolved 3.00 g of the compound represented by structural formula (17-c) and 1.89 g of the compound represented by structural formula (18-b). Hydrobromic acid (1.62 g, 47%) was dropped into the resulting solution, and the mixture was stirred at room temperature for 2 hours. After concentration under reduced pressure, the residue was extracted with 200 ml of chloroform and washed with water. The solution was separated, and then the solvent was evaporated off to give 4.15 g of the compound represented by structural formula (15-c).

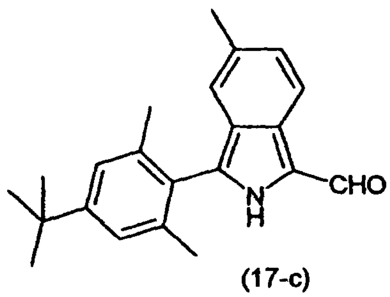

(17-c)

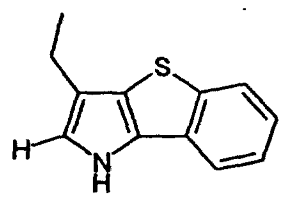

(18-b)

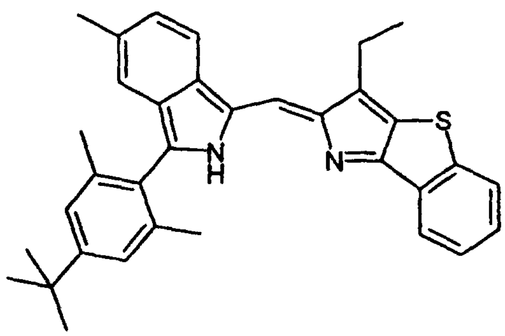

(15-c)

In the same manner, 6.00 g of the compound represented by structural formula (21-c) and 3.78 g of the compound represented by structural formula (18-b) was dissolved in 300 ml of ethanol. Hydrobromic acid (3.24 g, 47%) was dropped into the resulting solution, and the mixture was stirred at room temperature for 2 hours. After concentration under reduced pressure, the residue was extracted with 400 ml of chloroform and washed with water. The solution was separated, then the solvent was evaporated off to give 8.59 g of the compound represented by structural formula (16-c).

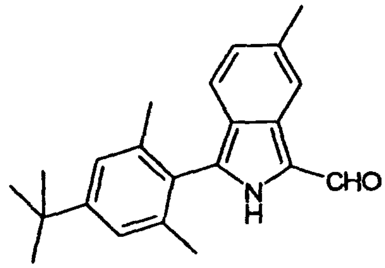

(21-c)

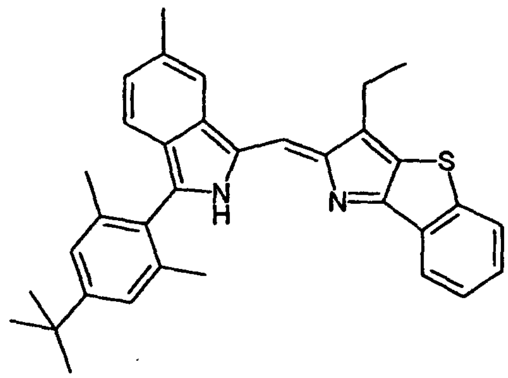

(16-c)

Then, in 400 g of ethanol, 4.00 g of the compound represented by structural formula (15-c) and 4.00 g of the compound represented by structural formula (16-c) were dissolved. After adding 2.89 g of copper acetate, the mixture was stirred at reflux temperature for 2 hours. After concentration under reduced pressure, the precipitate was collected by filtration and washed with methanol and water to give 7.55 g of the dipyrromethene-metal chelate compound mixture (m-43) represented by the structural formulas (3-43), (4-43) and (5-43).

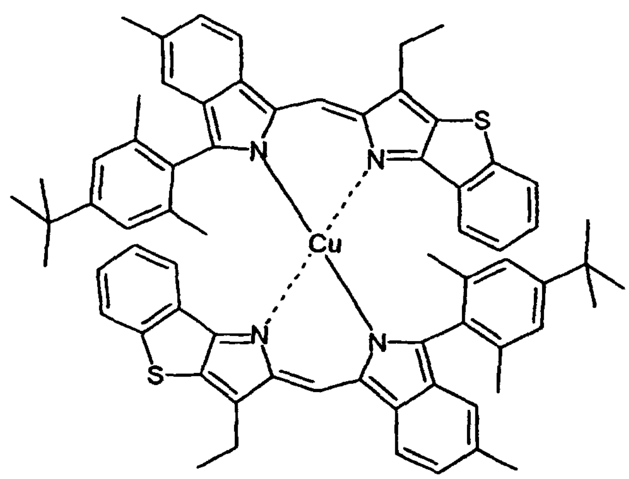

(3-43)

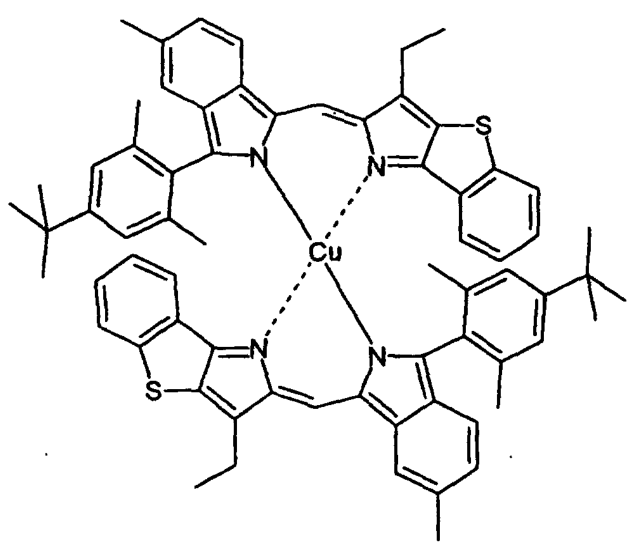

(4-43)

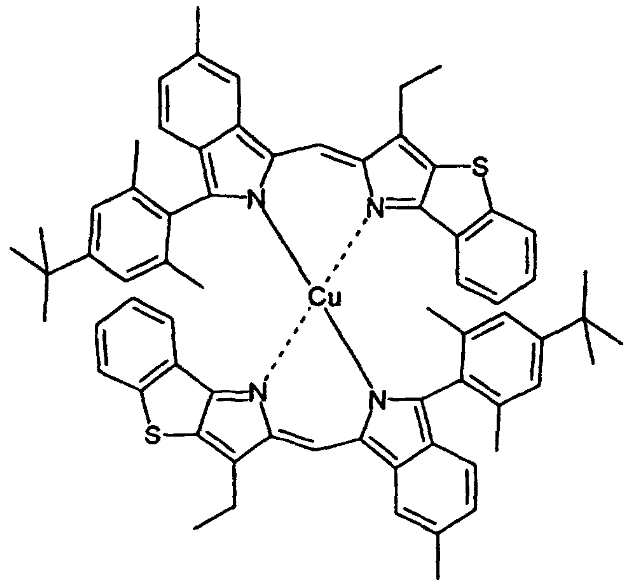

(5-43)

From the following analysis results, it was confirmed as the title compound.

| Elementary analysis: $C_{68}H_{66}N_4S_2Cu$ | | | |
|---|---|---|---|
| | C | H | N |
| Calculated (%) | 76.55 | 6.23 | 5.25 |
| Found (%) | 76.56 | 6.20 | 5.23 |
| MS (m/e): 1065 ($M^+$) | | | |

In addition, from HPLC analysis it was determined that the respective compounds had the following composition ratio.

| Compound (3-43) | Compound (4-43) | Compound (5-43) |
|---|---|---|
| 31% | 51% | 18% |

The compounds thus obtained exhibited in toluene a local absorption maximum at 598.0 nm and a gram absorption coefficient of 1.65 x $10^5$ ml/g.cm.

(Example 15) Preparation of a Dipyrromethene-metal Chelate Compound Mixture (Compound Mixture m-59)

In 150 ml of ethanol were dissolved 3.00 g of the compound represented by structural formula (17-c) and 2.00 g of the compound represented by structural formula (18-c). Hydrobromic acid (1.62 g, 47%) was dropped into the resulting solution, and the mixture was stirred at room temperature for 2 hours. After concentration under reduced pressure, the residue was extracted with 200 ml of chloroform and washed with water. The solution was separated, and then the solvent was evaporated off to give 4.50 g of the compound represented by structural formula (15-d).

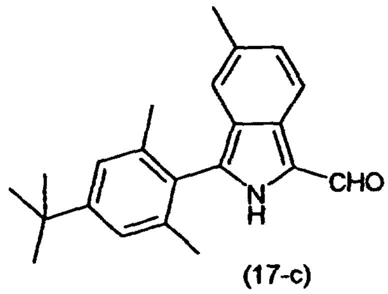

(17-c)

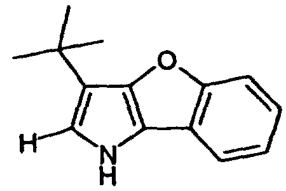

(18-c)

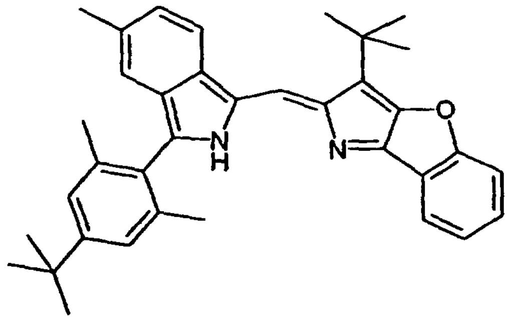

(15-d)

In the same manner, 6.00 g of the compound represented by structural formula (21-c) and 4.00 g of the compound represented by structural formula (18-c) was dissolved in 300 ml of ethanol. Hydrobromic acid (3.24 g, 47%) was dropped into the resulting solution, and the mixture was stirred at room temperature for 2 hours. After concentration under reduced pressure, the residue was extracted with 400 ml of chloroform and washed with water. The solution was separated, then the solvent was evaporated off to give 8.61 g of the compound represented by structural formula (16-d).

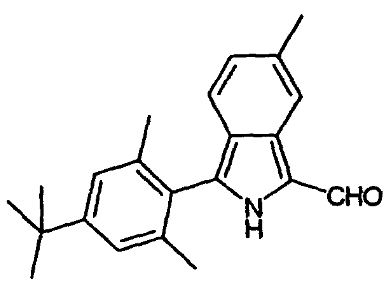

(21-c)

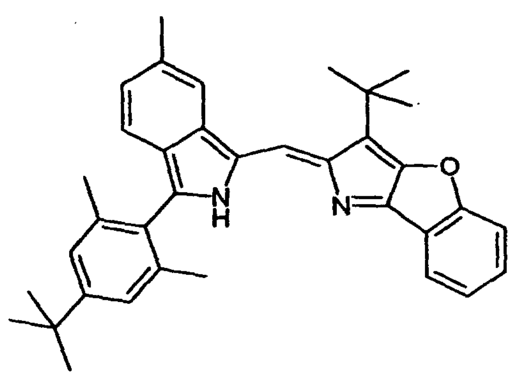

(16-d)

Then, in 400 ml of ethanol, 4.00 g of the compound represented by structural formula (15-d) and 6.00 g of

the compound represented by structural formula (16-d) were dissolved. After adding 3.50 g of copper acetate, the mixture was stirred at reflux temperature for 2 hours. After concentration under reduced pressure, the precipitate was collected by filtration and washed with methanol and water to give 9.22 g of the dipyrromethene-metal chelate compound mixture (m-59) represented by the structural formulas (3-59), (4-59) and (5-59).

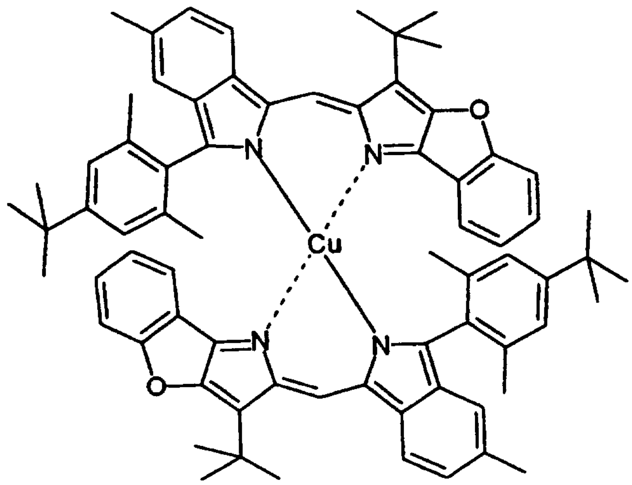

(3-59)

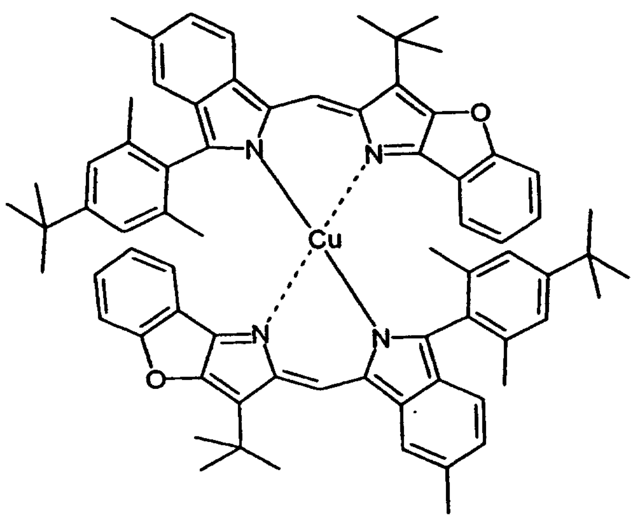

(4-59)

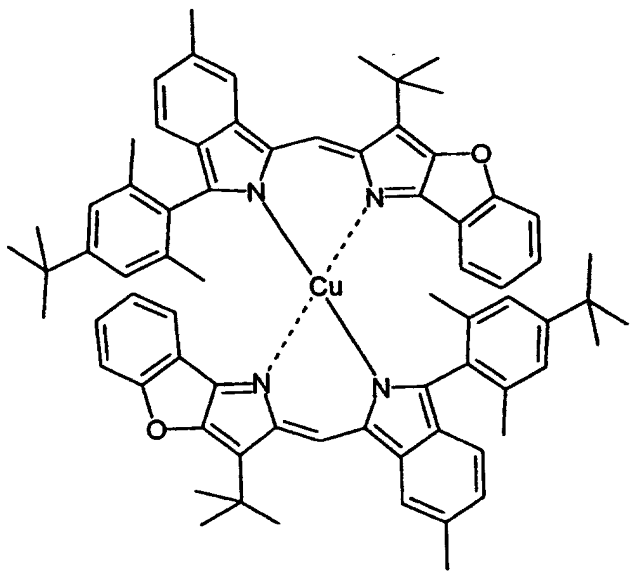

(5-59)

From the following analysis results, it was confirmed as the title compound.

| Elementary analysis: $C_{72}H_{74}N_4O_2Cu$ | | | |
|---|---|---|---|
| | C | H | N |
| Calculated (%) | 79.27 | 6.84 | 5.14 |
| Found (%) | 79.22 | 6.81 | 5.16 |
| MS (m/e): 1089 ($M^+$) | | | |

In addition, from HPLC analysis it was determined that the respective compounds had the following composition ratio.

| Compound (3-59) | Compound | Compound (5-59) |
|---|---|---|
| 32% | (4-59) 42% | 26% |

The compounds thus obtained exhibited in toluene a local absorption maximum at 589 nm and a gram absorption coefficient of 1.67 x $10^5$ ml/g.cm.

(Examples 17-27)

A dipyrromethene-metal chelate compound listed in Table 1, or a dipyrromethene-metal chelate compound mixture listed in Table 3, was suitably dissolved into a mixed solvent containing dimethylcyclohexane:cyclooctane 100: 8 (18 g/l) for depositing by spin coating so that the film thickness above the groove was adjusted to about 80 nm and the film thickness between grooves to 20 nm. After deposition, an optical recording medium was prepared in the same manner as Example 8.

The optical constant of 650 nm (refractive index, extinction coefficient) and the initial thermal weight loss temperature under nitrogen atmosphere for these recording layers of the optical recording medium (measured using a TA-50WS Shimadzu TG Analyzer) are shown in Table 5.

A DVDR compatible EFM+ signal was recorded onto this optical recording medium using a Pulstec Industrial Co., Ltd. disc tester DDU1000 at wavelength 661 nm, NA=0.60; linear velocity 3.5 m/s (DVD-R specifications recording speed) and linear velocity 14 m/s (DVD-R specification recording speed for 4x-speed). The recording conditions were a high-speed drive of respectively 1x-speed and 4x-speed having pulse conditions as described in the DVD-R Specification (version 2.0), and recording was carried out by making optimal adjustment to each pit. These recorded sites were measured for jitter at a DVD standard speed.

Table 5 shows the results of the measured jitter and the degree of modulation of 1x-speed and 4x-speed at

that time. For the DVD medium illustrated in the present example, a satisfactory jitter value over a broad power window was confirmed for both 1x-speed and 4x-speed.

Table-5

| | Compound Mixed Compound | Optical Properties | | TG Analysis | 1x-Speed Signal Characteristics | | 4x-Speed Signal Characteristics | |
|---|---|---|---|---|---|---|---|---|
| | | Refractive Index n | Extinction Coefficient k | Initial Thermal Weight Loss Temperature °C | Jitter % | Degree of Modulation | Jitter % | Degree of Modulation |
| Example 17 | 1-54 | 2.50 | 0.11 | 392 | 8.1 | 0.66 | 8.2 | 0.72 |
| Example 18 | 1-60 | 2.55 | 0.16 | 403 | 8.1 | 0.62 | 8.0 | 0.70 |
| Example 19 | 1-67 | 2.51 | 0.10 | 398 | 7.6 | 0.65 | 7.5 | 0.69 |
| Example 20 | 1-77 | 2.60 | 0.14 | 390 | 8.0 | 0.67 | 7.7 | 0.72 |
| Example 21 | m-5 | 2.58 | 0.09 | 360 | 7.9 | 0.61 | 8.1 | 0.68 |
| Example 22 | m-41 | 2.71 | 0.15 | 357 | 6.9 | 0.63 | 7.9 | 0.75 |
| Example 23 | m-43 | 2.69 | 0.12 | 372 | 6.8 | 0.63 | 6.7 | 0.72 |
| Example 24 | m-50 | 2.66 | 0.10 | 389 | 7.5 | 0.60 | 8.2 | 0.67 |
| Example 25 | m-59 | 2.55 | 0.13 | 402 | 7.6 | 0.64 | 6.9 | 0.67 |
| Example 26 | m-60 | 2.59 | 0.15 | 415 | 7.9 | 0.62 | 7.0 | 0.69 |
| Example 27 | m-61 | 2.60 | 0.16 | 396 | 7.6 | 0.65 | 7.6 | 0.70 |

Industrial Applicability

Using the dipyrromethene-metal chelate compound according to the present invention, wherein the initial principal weight loss temperature according to thermogravimetric analysis is from 330°C or more to 500°C or less, as a recording layer allows the provision of a recordable optical recording medium that is suitable for high-speed high-density recording.

## Claims

1. An optical recording medium comprising at least a recording layer and a reflecting layer on a transparent substrate having a guide groove formed thereon, wherein the recording layer contains at least one dipyrromethene-metal chelate compound of which the initial principal weight loss temperature is thermogravimetrically from 330°C through 500°C inclusive.

2. The optical recording medium according to claim 1, wherein the dipyrromethene-metal chelate compound is represented by general formula (1),

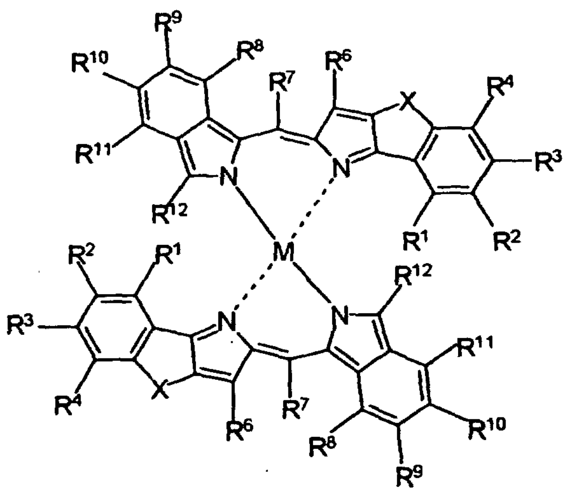

(1)

wherein $R^1$ to $R^4$ and $R^6$ to $R^{11}$ each independently represent hydrogen, halogen, optionally substituted alkyl, alkoxy, aryl, aryloxy or a substituent represented by formula (a),

$$-CO-R^{13} \quad (a)$$

wherein $R^{13}$ represents optionally substituted alkyl, optionally substituted aralkyl or optionally substituted aryl; $R^{12}$ represents optionally substituted aryl; X represents -O-, -S- or -CH($R^5$)-; $R^5$ represents hydrogen, halogen, optionally substituted alkyl, alkoxy, or aryl; and M represents a transition element.

3. The optical recording medium according to claim 2, wherein the dipyrromethene-metal chelate compound is represented by general formula (2),

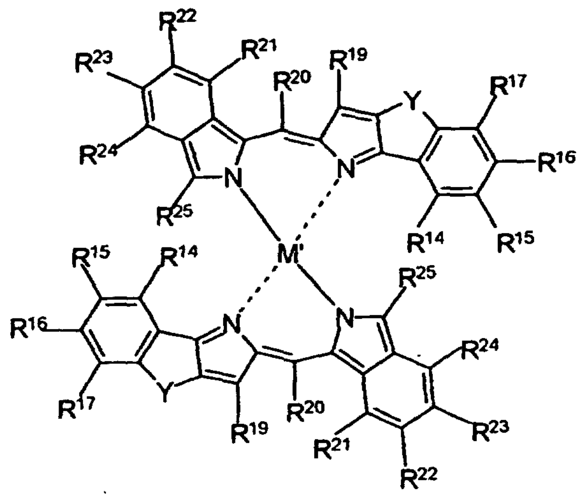

(2)

wherein $R^{14}$ to $R^{17}$ and $R^{19}$ to $R^{24}$ each independently represent hydrogen, halogen, optionally substituted alkyl, alkoxy, aryl or aryloxy; $R^{25}$ represents optionally substituted aryl; Y represents -O-, -S- or -CH($R^{18}$)-; $R^{18}$ represents hydrogen, halogen, optionally substituted alkyl, alkoxy or aryl; and M' represents a transition element.

**4.** The optical recording medium according to claim 1 comprising a mixture of dipyrromethene-metal chelate compounds represented by general formulas (3), (4) and (5),

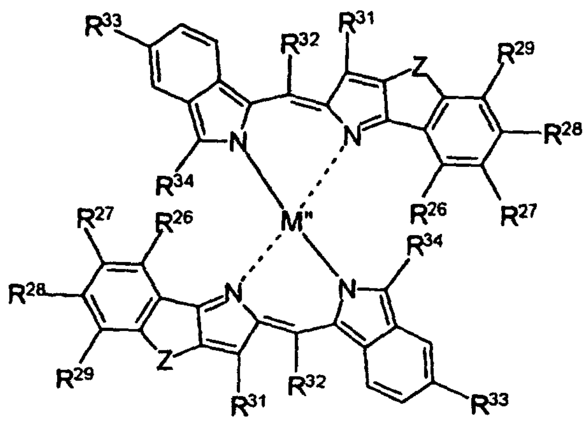

(3)

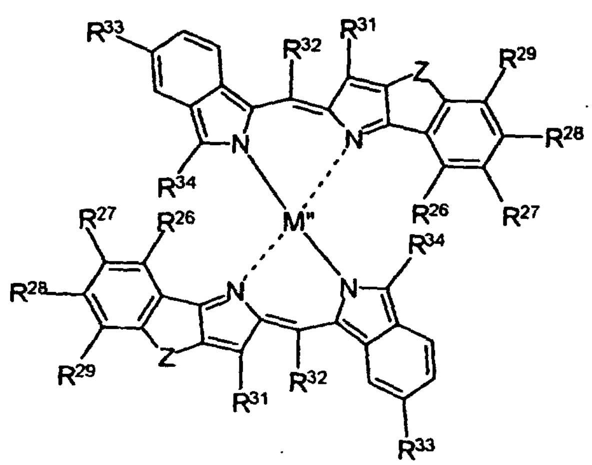

(4)

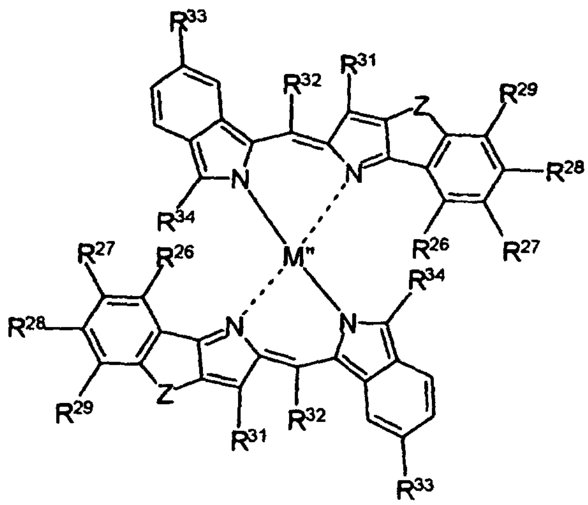

(5)

wherein $R^{26}$ to $R^{29}$ and $R^{31}$ to $R^{32}$ each independently represent hydrogen, halogen, optionally substituted alkyl, alkoxy, aryl, aryloxy or a substituent represented by formula (b),

$$-CO-R^{35} \qquad (b)$$

wherein $R^{35}$ represents optionally substituted alkyl, optionally substituted aralkyl or optionally substituted aryl; $R^{33}$ represents halogen, optionally substituted alkyl, alkoxy, aryl or aryloxy; $R^{34}$ represents optionally substituted aryl; Z represents oxygen, sulfur or CH-$R^{30}$; $R^{30}$ represents hydrogen, halogen, optionally substituted alkyl, alkoxy, or aryl; and M" represents a transition element.

**5.** A dipyrromethene-metal chelate compound represented by general formula (6),

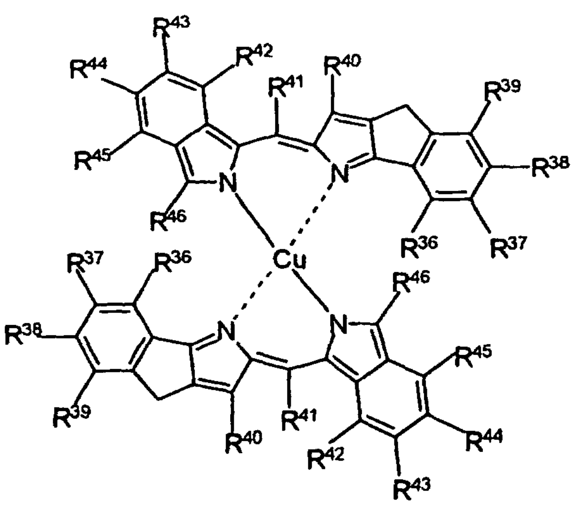

(6)

wherein $R^{36}$ to $R^{39}$ and $R^{41}$ to $R^{45}$ each independently represent hydrogen, halogen, optionally substituted alkyl, alkoxy, aryl, aryloxy; $R^{40}$ represents optionally substituted alkyl having a total number of from 2 to 12 carbon atoms; and $R^{46}$ represents optionally substituted aryl.

**6.** A mixture of dipyrromethene-metal chelate compounds represented by general formulas (7), (8) and (9),

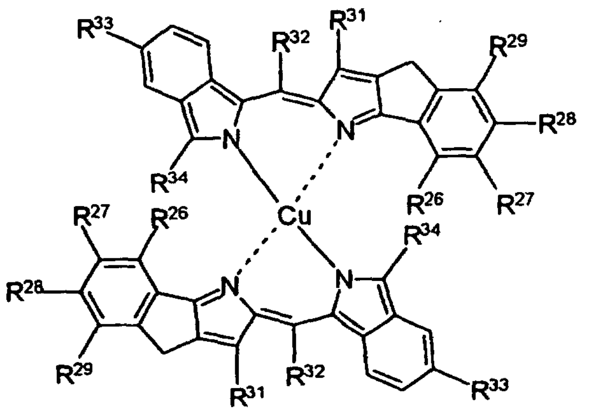

(7)

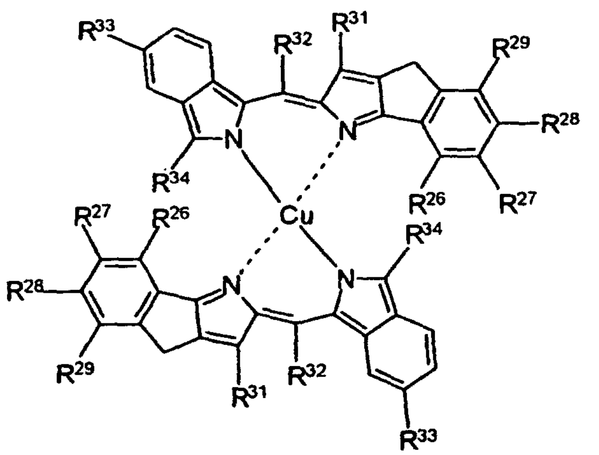

(8)

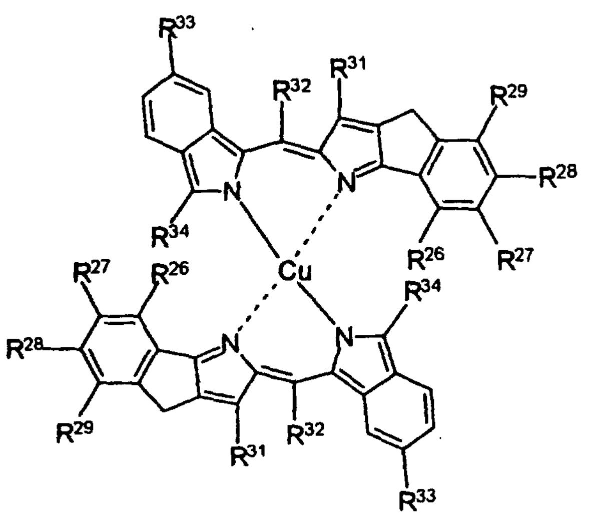

(9)

wherein $R^{26}$ to $R^{29}$ and $R^{31}$ to $R^{32}$ each independently represent hydrogen, halogen, optionally substituted alkyl, alkoxy, aryl, aryloxy or a substituent represented by formula (b),

$$-CO-R^{35} \qquad (b)$$

wherein $R^{35}$ represents optionally substituted alkyl, optionally substituted aralkyl or optionally substituted aryl

$R^{33}$ represents halogen, optionally substituted alkyl, alkoxy, aryl or aryloxy; and $R^{34}$ represents optionally substituted aryl.

Figure 1

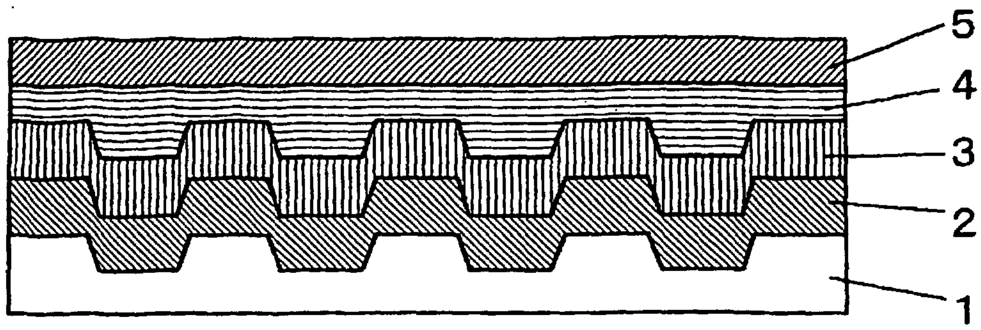


Figure 2

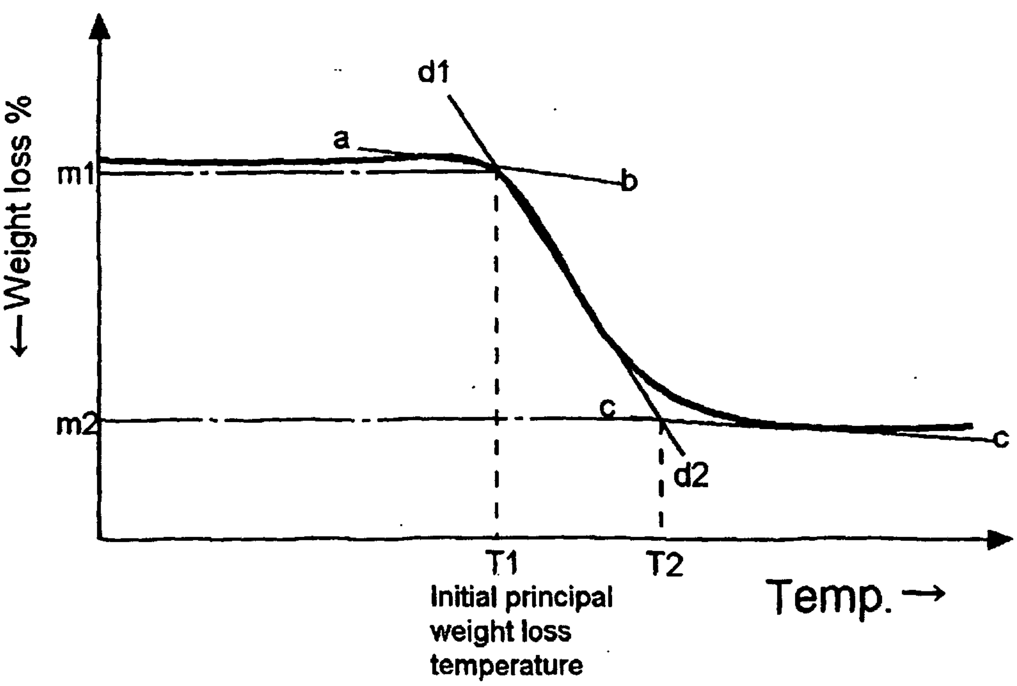

## INTERNATIONAL SEARCH REPORT

International application No.
PCT/JP03/03840

**A. CLASSIFICATION OF SUBJECT MATTER**

Int.Cl[7] B41M5/26, C09B23/04, C07D209/70, C07D491/048, C07D495/04, G11B7/24

According to International Patent Classification (IPC) or to both national classification and IPC

**B. FIELDS SEARCHED**

Minimum documentation searched (classification system followed by classification symbols)

Int.Cl[7] B41M5/26, C09B23/04, C07D209/70, C07D491/048, C07D495/04, G11B7/24

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)

CAPLUS(STN), REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| X | EP 822544 A (MITSUI CHEMICALS INC., YAMAMOTO CHEMICALS INC.), 04 February, 1998 (04.02.98), Full text; all drawings; particularly, tables 1 to 11 & JP 10-226172 A | 1 |
| X | JP 11-256057 A (MITSUI CHEMICALS INC., YAMAMOTO CHEMICALS INC.), 21 September, 1999 (21.09.99), Full text; all drawings (Family: none) | 1 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 June, 2003 (19.06.03) | 01 July, 2003 (01.07.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/03840

C (Continuation). DOCUMENTS CONSIDERED TO BE RELEVANT

| Category* | Citation of document, with indication, where appropriate, of the relevant passages | Relevant to claim No. |
|---|---|---|
| X | JP 11-302253 A (MITSUI CHEMICALS INC., YAMAMOTO CHEMICALS INC.), 02 November, 1999 (02.11.99), Full text; all drawings (Family: none) | 1 |
| P,X | US 2002/48646 A1 (Ricoh Co., Ltd.), 25 April, 2002 (25.04.02), Full text; all drawings & JP 2002-288841 A | 1 |
| P,X | EP 1130584 A2 (MITSUI CHEMICALS INC., YAMAMOTO CHEMICALS INC.), 05 September, 2001 (05.09.01), Full text; all drawings & JP 2002-212456 A | 1-3,5 |
| P,X | JP 2003-73574 A (MITSUI CHEMICALS INC., YAMAMOTO CHEMICALS INC.), 12 March, 2003 (12.03.03), Full text; all drawings (Family: none) | 1-6 |
| A | EP 903733 A (MITSUI CHEMICALS INC., YAMAMOTO CHEMICALS INC.), 24 March, 1999 (24.03.99), Full text; all drawings (Family: none) | 1 |

Form PCT/ISA/210 (continuation of second sheet) (July 1998)

**INTERNATIONAL SEARCH REPORT**

International application No.
PCT/JP03/03840

**Box I Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

(See extra sheet)

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☒ No protest accompanied the payment of additional search fees.

Form PCT/ISA/210 (continuation of first sheet (1)) (July 1998)

INTERNATIONAL SEARCH REPORT

International application No.
PCT/JP03/03840

Continuation of Box No.II of continuation of first sheet(1)

1. Claims 1-3 relate to an optical recording medium containing in the recording layer a dipyrromethene compound exhibiting a main weight loss initiating temperature of 330 to 500°C in the thermogravimetric analysis.
2. Claim 4 relates to an optical recoding medium containing in the recording layer a mixture of dipyrromethene-metal chelate compounds represented by the general formulae (3), (4) and (5) and exhibiting a main weight loss initiating temperature of 330 to 500°C in the thermogravimetric analysis.
3. Claim 5 relates to dipyrromethene-metal chelate compounds represented by the general formula (6) in claim 5.
4. Claim 6 relates to a mixture of dipyrromethene-metal chelate compounds represented by the general formulae (7), (8) and (9) in claim 6.

In the above groups 1-4 of inventions, "dipyrromethene-metal chelate compounds", "an optical recording medium containing in the recording layer a dipyrromethene-chelate compound exhibiting a main weight loss initiating temperature of 330 to 500 °C in the thermogravimetric analysis" "a mixture of dipyrromethene-chelate compounds represented by the general formulae (3), (4) and (5) in claim 4" and "dipyrromethene-metal chelate compounds represented by the general formula (6) in claim 5", which are thought to be special technical features within the meaning of PCT Rule 13.2, are disclosed in EP 822544 A1 (MITSUITOATSU CHEMICALS INCORPORATED, YAMAMOTO CHEMICALS INC.), EP 1130584 A2 (MITSUI CHEMICALS INC., YAMAMOTO CHEMICALS INC.), and JP 2003-73574 A (MITSUI CHEMICALS INC., YAMAMOTO CHEMICALS INC.), being known constitutions. Thus, the groups of inventions are not considered as relating to a group of inventions so linked as to form a single general inventive concept (No description on the inventions of claims 4 and 6 can be found in the description or drawings originally attached to the request of Japanese Patent Application No. 2002-96351 or 2002-290155).

The above groups of inventions do not have such a relationship as to satisfy the unity of invention.